(19)

Europäisches
Patentamt
European
Patent Office
Office européen
des brevets

(11) **EP 3 812 765 A1**

(12) **EUROPEAN PATENT APPLICATION**

(43) Date of publication:
**28.04.2021  Bulletin 2021/17**

(51) Int Cl.:
**G01N 33/50** *(2006.01)*  **G01N 35/00** *(2006.01)*
**G06K 9/00** *(2006.01)*  **G06T 7/00** *(2017.01)*

(21) Application number: **19204932.8**

(22) Date of filing: **23.10.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
**KH MA MD TN**

(71) Applicants:
• **Deutsches Krebsforschungszentrum,
Stiftung des öffentlichen Rechts
69120 Heidelberg (DE)**

• **Berliner Institut für Gesundheitsforschung
Zentrum
Digitale Gesundheit
10117 Berlin (DE)**

(72) Inventors:
• **Kallenberger, Stefan
69120 Heidelberg (DE)**
• **Treis, Tim
69120 Heidelberg (DE)**
• **Di Ponzio, Chiara
69120 Heidelberg (DE)**
• **Eils, Roland
12555 Berlin (DE)**

(54) **METHOD AND SYSTEM FOR CLOSED-LOOP LIVE-CELL IMAGING**

(57)     The invention refers to a live-cell imaging method for acquiring experimental data of one or more biological probes (20) comprising: obtaining, by an imaging device (12), at least one optical measurement of the one or more biological probes (20); determining, by a processing module (30), at least one measurement value of a cellular parameter of the one or more biological probes from the at least one optical measurement; determining, by the processing module (30), whether the at least one measurement value satisfies a convergence criterion of a regulatory task; and applying, by a probe manipulation device (16), at least one experimental stimulus to the one or more biological probes (20) according to the regulatory task, if the convergence criterion is not fulfilled, wherein the experimental stimulus sets an environmental condition of the biological probe to which the experimental stimulus is applied, wherein the regulatory task defines a target evolution of the one or more biological probes (20). The invention further refers to a corresponding live-cell imaging system (10), to a processing module (30) configured for controlling the live-cell imaging system, and to a corresponding digital storage device.

Fig. 1

**Description**

FIELD OF THE INVENTION

The present invention is in the field of experimental data acquisition. In particular, the present invention relates to a live-cell imaging method and a corresponding system for acquiring experimental data of one or more biological probes.

BACKGROUND OF THE INVENTION

Data acquisition in live-cell imaging methods typically comprises a sequence of data collection, data processing and data evaluation processes and requires monitoring or direct intervention by a skilled human operator.

Existing live-cell imaging systems allowing for some degree of automatization are very costly systems requiring large amounts of space.

Biological models are broadly used for data acquisition. However, parameter estimation within the context of the biological models can introduce sources of error that may negatively affect the efficiency and accuracy of the data evaluation process.

Therefore, there is room for technical improvement regarding live-cell imaging, in particular with respect to automatisation and modelling within the context of live-cell imaging.

SUMMARY OF THE INVENTION

The present invention relates to a live-cell method according to claim 1, a live-cell imaging system according to claim 9, a processing module according to claim 13, and a digital storage device according to claim 15. Preferred embodiments of the invention are defined in the appended dependent claims.

One aspect of the invention concerns a live-cell imaging method for acquiring experimental data of one or more biological probes. The one or more biological probes may comprise a plurality of living and/or dead cells, for example cells of a tissue or a biological system. The one or more biological probes may comprise a plurality of groups of cells, wherein each group of cells may be arranged in a corresponding cell container, such as a petri dish or a well of a multi-well plate.

The method comprises obtaining, by an imaging device, at least one optical measurement of the one or more biological probes. The imaging device may be an optical device for obtaining optical measurements of the one or more biological probes, in particular optical measurements in the micrometre range. The imaging device may comprise a microscope, and/or a camera for obtaining the optical measurements. The one or more biological probes may be arranged with respect to the imaging device such that they are optically accessible by the imaging device. For example, the one or more biological probes may be arranged on a transparent plate and the imaging device may be configured to obtain the optical measurements through the transparent plate.

In further examples, the plate needs not be transparent, in particular if the imaging device is configured for optically accessing the one or more biological probes from a direction opposite to the plate, e.g., from above. The one or more biological probes may be arranged on different plates, wherein the plates may be stacked over each other in a vertical direction, with the imaging device being movable in said vertical direction.

The at least one optical measurement may be obtained according to a predefined measurement routine comprising a sequence of measuring events, wherein each measuring event may be defined by a measuring position corresponding to one of the biological probes and/or a measuring time of the measurement. For example, for biological probes $P_1$ to $P_n$, may be located on a transparent top plate, such as a glass plate, at positions characterised by coordinates $(x_1, y_1)$, $(x_2, y_2)$, ... , $(x_n, y_n)$ on the plane of the plate, such that the biological probe $P_i$ is optically accessible by the imaging device in optimal measuring conditions when the imaging device is positioned in a location defined by the corresponding coordinates $(x_i, y_i)$, for example below the biological probe $P_i$. The measurement routine may specify that an optical measurement be obtained from $P_1$ at position $(x_1, y_1)$ at a time $t_1$, an optical measurement be obtained from $P_2$ at position $(x_2, y_2)$ at a time $t_2$ and so on, and finally that an optical measurement be obtained from $P_n$ at position $(x_n, y_n)$ at a time $t_n$. The measurement routine may specify the same or different image acquisition settings for different measuring events, such as a number of optical measurements for each biological probe, and overall number of optical measurements and/or an image resolution. For example, the measuring routine may define that 30 optical measurements be obtained from each of the biological probes at a resolution of 8 megapixels at time intervals of 60 seconds between successive optical measurements.

The method of the invention further comprises determining, by a processing module, at least one measurement value of a cellular parameter of the one or more biological probes from the at least one optical measurement. The processing module may be functionally connected or connectable to the imaging device and may be configured for receiving the one or more optical measurements as an input. The processing module may be located proximate to the imaging device and may be connectable to the imaging device via local connection means such as wiring or short-range wireless communication means, for example WLAN or Bluetooth. However, the processing module may be located remotely from the imaging device and may be connectable to the imaging device via long-range wired or wireless connection means, such as fiber optic, radio communication, or an internet connection. The processing module may be configured for transforming the optical information of the one or more optical measurements, received as an input, into electronic signals for electronically processing and analysing said optical information, for example by a processor, which may be included in the processing module.

"Cellular parameter" refers herein to a measurable physical, chemical or biological property of the one or more biological probes, such as any of cell number, number or fraction of living cells, number or fraction of dead or dying cells (i.e. number or fraction of apoptotic cells), cell proliferation rate, cell death rate, cell division rate, cell differentiation rate, cell exocytosis rate, cell endocytosis rate, cell size, cell dimensions, cell adherence area, and beating frequency, cell depolarization rate and a drug concentration. "Measurement value" refers herein to a quantity, which can be measured or obtained, for example computed, from an optical measurement of a cellular parameter. For example, upon measuring cell number, a measurement value of "10" may be obtained for the cellular parameter "cell number".

Determining, by the processing module, the measurement value of the cellular parameter of the one or more biological probes may comprise classifying, counting and/or identifying cells of the one or more biological probes with respect to the cellular parameter. This may for example correspond to a quantification of the measurement value or to a binary evaluation, for example classifying the cells as living or dead cell, or to a classification of the cells into one of a plurality of states or labels, such as "small size", "medium size" and "large size".

The cells in the one or more biological probes may be classified by an artificial intelligence algorithm, for example a neural network algorithm, trained for classifying, counting and/or identifying cells of the one or more biological probes with respect to the cellular parameter based on an optical measurement or a plurality of optical measurements. For this purpose, the processing module may comprise an artificial intelligence algorithm trained for extracting measurement values of the cellular parameter from the optical measurements, for example for extracting a number of living and/or dead cells from an optical image of the cells. Additionally or alternatively, the processing module may comprise or be connected or connectable to a processor comprising such an algorithm.

The method further comprises, determining, by the processing module, whether the at least one measurement value satisfies a convergence criterion of a regulatory task. "Regulatory task" refers herein to a rule defining a target evolution of the one or more biological probes. "Convergence criterion" refers herein to a condition to be fulfilled or maintained for considering that the one or more biological probes are behaving or evolving according to the aforesaid target evolution, i.e. that the regulatory task is complied with. For example, it may be considered, in some embodiments, that the one or more biological probes have not completed the target evolution according to the regulatory task as long as the convergence criterion is not fulfilled.

The method further comprises applying, by a probe manipulation device, at least one experimental stimulus to the one or more biological probes according to the regulatory task, if the convergence criterion is not fulfilled. The experimental stimulus sets, i.e. influences so as to determine, an environmental condition of the corresponding biological probe or probes to which the experimental stimulus is applied. The probe manipulation device is a device configured for influencing the value of one or physical properties of the one or more biological probes, in particular the cellular parameter, by varying an environmental condition of the biological probes. "Environmental condition" refers herein to the physical, chemical and biological conditions to which a probe is exposed, for example temperature, concentration of a drug, or exposure to electromagnetic radiation.

Applying the at least one experimental stimulus to a biological probe may comprise varying a physical condition to which said probe is exposed, wherein the physical condition may be at least one of a light intensity, a perfusion rate and/or a concentration of an environmental fluid, a temperature, exposure to an interacting agent, such as proteins, ions, or nutrients, an electrical voltage and/or a magnetic field.

As long as the convergence criterion is not fulfilled, the probe manipulation device may influence the one or more biological probes by means of corresponding experimental stimuli "according to the regulatory task", i.e. such that the one or more biological probes follow or at least approach, driven by the experimental stimuli provided by the probe manipulation device, the target evolution defined by the regulatory task.

The regulatory task may define a target value of the cellular parameter of the biological probe to which the experimental stimulus is applied. In this case, applying the experimental stimulus to the one or more biological probes "according to the regulatory task" may imply providing an experimental stimulus that makes the corresponding biological probe evolve towards or maintain a situation in which the cellular parameter takes or at least approaches the target value, i.e. in which the measurement value corresponds to the target value or is at least closer to the target value as compared to the situation before applying the experimental stimulus. For example, the regulatory task may define a target division rate of the one or more biological probes.

The convergence criterion may then be fulfilled when the determined measurement value of the cellular parameter corresponds to the target value of the cellular parameter defined by the regulatory task within a predefined tolerance and/or when this correspondence is maintained in time. For example, if the cellular parameter is a number of living cells and the regulatory task corresponds to a given number of living cells, such as $10^4$, the convergence criterion may be fulfilled when and/or as long as the measurement value of the number of cells determined by the analysis unit corresponds to said given number of living cells within the predefined tolerance, for example to $10^4 \pm 10^2$.

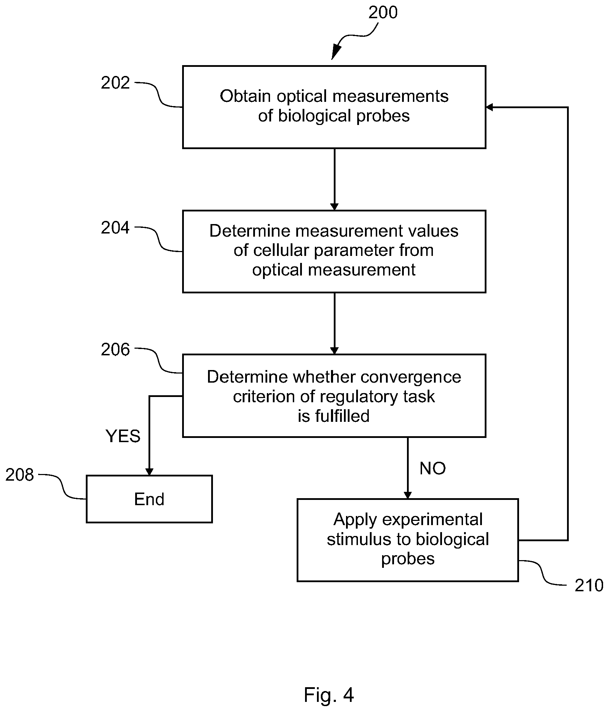

In some preferred embodiments, the regulatory task may define the target value of the cellular parameter such that the cellular parameter or a derivative thereof is kept constant, for example such that the cell division rate corresponds to a predefined value or such that the cell division rate is maximal or minimal (i.e. its derivative is zero). In some preferred embodiments, the regulatory task may define the target value of the cellular parameter such that the cellular parameter follows a predefined parameter trajectory or time-dependent function, for example a predefined time evolution. Additionally or alternatively, the regulatory task may define the target value of the cellular parameter such that a biological process depending on the cellular parameter of the corresponding probe achieves a target state, for example a desired extremal state, such as a state of maximal cell division rate or a state of maximal fraction of cells differentiating into a certain cell type. An example of a related method for acquiring experimental data is provided below with respect to the embodiment illustrated in Fig. 4.

Additionally or alternatively, the regulatory task may define a target environmental condition of the biological probe to which the experimental stimulus is applied. In this case, applying the experimental stimulus to the one or more biological probes "according to the regulatory task" may imply providing an experimental stimulus that makes the environmental condition of the corresponding biological probe or probes evolve towards the target environmental condition. For example, the regulatory task may define a stimulus trajectory, wherein the stimulus trajectory determines a sequence of experimental stimuli to be applied to the one or more biological probes, for example a sequence of experimental stimuli defined as evaluations of a time-dependent stimulus function at given times.

As a further example, the experimental stimulus may correspond to a substance concentration, such as an inhibitor concentration, and the stimulus trajectory may correspond to a sequence of substance concentrations to be applied, for instance to be sequentially applied in time to a given one of the one or more biological probes in a predefined time sequence, or to be simultaneously applied to different biological probes.

The method of the invention hence allows defining an automated closed-loop process for image acquisition of experimental data of the one or more biological probes with stimulus feedback in real time to guide the evolution of the biological probes during image acquisition, such as to control the dynamical behaviour of the one or more biological probes in a predefined manner, i.e. according to the regulatory task. Thereby, experimental automatization can be achieved in an easy and controlled manner, allowing for a plethora of applications, some examples of which will be explained in detail below. The method of the invention may allow obtaining large amounts of experimental data without requiring active intervention or monitoring by a human operator.

The previously described method steps of i) obtaining the at least one optical measurement, ii) determining the at least one measurement value from the at least one optical measurement, iii) determining whether the convergence criterion of the regulatory task is satisfied, and iv) applying the at least one experimental stimulus may be executed in any order and may be cyclically repeated in any order.

According to some preferred embodiments, the method may further comprise recording a sequence of measurement values and associated environmental conditions corresponding to a number of cyclic repetitions of steps of i), ii) and, iv). The aforesaid cyclic repetitions may optionally comprise step of iii), wherein step iii) may follow or precede any of the aforementioned steps i), ii), and iv).

Before or after step iv), the method may further comprise fitting one or more model parameters of a biological model for the one or more biological probes to the sequence of measurement values and associated environmental conditions. "Associated environmental conditions" are environmental conditions of a biological probe when the associated optical measurement of said biological probe is obtained. The biological model may provide estimated values of the cellular parameter of the one or more biological probes as a function of the corresponding environmental condition and of the model parameters. The fitting of the one or more model parameters may for example comprise a least square fitting, Bayesian interference and/or log-likelihood maximisation.

The biological model may be defined by a set of functions (for example constraints) and/or equations, for example by a set of differential equations, defining a relation between values of the cellular parameter of the one or more biological probes and the environmental condition to which a respective biological probe is exposed as a function of the model parameters. For example, the biological model may model cell apoptosis by relating a number of dead cells to a concentration of one or more reactants, as defined by a series of model parameters, for instance a series of kinetic parameters of the model. Initial values of the model parameters may be initially approximated by an educated guess or a random guess.

By recording a sequence of measurement values and associated environmental conditions and then fitting the one or more model parameters to the sequence, the confidence level of the model parameters may be improved with respect to an initial guess or to a pre-existing approximation or fit. The method of the invention can thereby implement online learning to improve model fitting during image acquisition.

In some embodiments, the aforesaid number of cyclic repetitions may be 1, 2, 5, 10, 15, 25, 50, 100, or 1000. This means that method steps i) and ii) or i) to iii) or i) to iv) may be repeated (in any order) 1, 2, 5, 10, 15, 25, 50, 100, or 1000 times before the model parameters are fitted or refitted. For example, if the number of cyclic repetitions is 10, an initial guess of the one or more model parameters for at least one of the one or more biological cells may be improved by fitting the model parameters based on the first 10 measurement values that have been measured (and possibly associated experimental conditions) to obtain a first fit. The first fit may be refitted each time a new measurement value has been measured, i.e. based on a set of 11 measurement values to obtain a second fit, then based on a set of 12 measurement values to obtain a third fit and so on. However, the first fit may additionally or alternatively be refitted after a number of measurement values corresponding to the aforesaid number of cyclic repetitions, in this exemplary case 10, have been measured, i.e. based on a set of 20 measurement values to obtain a second fit, then based on a set of 30 measurement values to obtain a third, and so on. The cyclic repetitions may be set forth as long as the convergence criterion of the regulatory task is not fulfilled, for a predefined number of times, or until a predefined stimulus trajectory is finalised.

According to some embodiments, fitting the one or more model parameters of the biological model may further comprise determining an updated stimulus trajectory and applying to at least one of the one or more biological probes, by the probe manipulation device, at least one of the experimental stimuli of the updated stimulus trajectory. The updated stimulus trajectory determines a sequence of updated experimental stimuli. The updated stimulus trajectory may be determined such that a covariance of the one or more model parameters as a function of the stimulus trajectory is minimised. Thereby, confidence intervals of the one or more model parameters can be minimized to reduce an uncertainty of estimated values thereof. For example, the stimulus trajectory may be defined as a time-dependent function characterised by a set of stimulus parameters. The aforesaid covariance of the one or more model parameters may then be determined such that a covariance of the one or more model parameters as a function of the stimulus parameters be minimised. The covariance of the model parameters may be determined as a function of a Fisher information matrix defined for the biological model as a function of the model parameters, as will be explained in further detail below (cf. Example 1).

According to these embodiments, a first (current) stimulus trajectory may be defined for obtaining a series of optical measurements of the one or more biological probes and the series of optical measurements may be used for fitting one or more model parameters of a biological model as previously explained. An optimisation problem may then be defined for the model parameters with respect to the stimulus trajectory, for example with respect to the stimulus parameters, in order to determine the updated stimulus trajectory. The updated stimulus trajectory may then be added to or may replace said first stimulus trajectory, i.e. may take the place, for example in a volatile memory of a processing module, of the stimulus trajectory to be applied to the biological probes by the probe manipulation device.

The updated stimulus trajectory may then be applied to the one or more biological probes by the probe stimulation device for a new series of optical measurements of the cellular parameter of the one or more biological probes. The new series of optical measurements may then be used for refitting the one or more model parameters with improved accuracy with respect to the foregoing fitting or refitting. Since the updated stimulus trajectory is determined by solving an optimisation problem with respect to the stimulus trajectory to minimise the covariance of the model parameters, the model parameters obtained from this new fitting when applying the updated stimulus trajectory have a reduced covariance in comparison to the previously determined (fitted) model parameters. As a consequence, the accuracy of the biological model, as defined by the refitted model parameters, is improved.

Once the refitted model parameters have been determined based on a series of optical measurements for the updated stimulus trajectory, the process of determining a further updated stimulus trajectory and refitting the model parameters may be repeated for a predefined number of cycles or until a fitting convergence criterion is fulfilled. For example, in the event of having one model parameter, the process of determining a further updated stimulus trajectory and refitting the model parameters may be repeated until a covariance of the model parameter does not vary between subsequent refittings by more than a predefined threshold. In the event of having more than one model parameter, the process of determining a further updated stimulus trajectory and refitting the model parameters may be repeated until the trace or the determinant of a covariance matrix of the model parameters does not vary between subsequent refittings by more than a predefined threshold.

Applying the updated stimulus trajectory to the one or more biological probes as previously described allows adapting the environmental condition of the one or more biological probes, by means of the applied experimental stimuli, so as to optimise the accuracy of the biological model, for example by minimising a covariance value or values of the model parameters, for example a covariance of one model parameter or a parameter related to a covariance matrix of more than one model parameters, in particular a determinant or a trace, as previously explained. The experimental conditions of the one or more biological probes are thereby optimised for obtaining a higher accuracy for the biological model in a more efficient manner, as compared to a situation with no such closed-loop control of the experimental conditions.

Accordingly, the method of the present invention may provide a way of autonomously (i.e. in a closed-loop manner without requiring human supervision in real time) optimising experimental conditions with regard to a particular dynamical evolution of the one or more biological probes, for example such that the cellular parameter or a derivative thereof corresponds to a predefined value, function or trajectory. Further, the method of the present invention may additionally provide a way of autonomously optimising experimental conditions for the definition or fitting of a biological model for the one or more biological probes. An example of a related method for acquiring experimental data is provided below in Example 1.

In some embodiments of the invention, fitting the one or more model parameters of the biological model may comprise, additionally or alternatively, determining a subsequent experimental stimulus of the stimulus trajectory for setting a "significant environmental condition" of the one or more biological probes, wherein the significant environmental condition has a value greater than a first environmental condition and smaller than a second environmental condition. The first environmental condition is set by a first experimental stimulus of the stimulus trajectory and the second environmental condition is set by a second experimental stimulus of the stimulus trajectory. The first and second environmental conditions are defined as the environmental conditions set by the applied stimulus trajectory between which a variation of the cellular parameter as a function of the environmental condition is extremal, i.e. maximal or minimal, with respect to the environmental conditions set by the stimulus trajectory. A relative variation of the cellular parameter as a function of the environmental condition between the first and second environmental conditions may be greater or smaller than all other relative variations of the cellular parameter defined at any two environmental conditions set by corresponding experimental stimuli of the applied stimulus trajectory, in particular set by any two consecutive experimental stimuli of the applied stimulus trajectory.

In some embodiments, the subsequent experimental stimulus may be determined as a function of the first and second experimental stimuli, for example as a mean point function, a weighted average, a logarithmic mean point function of the first and second experimental stimuli. In some embodiments, the subsequent experimental stimulus may be set to a value that minimizes an optimisation parameter, wherein the optimisation parameter may correspond to a covariance value of a model parameter, if the biological model is based on one model parameter, or to the trace or the determinant of a covariance matrix of the model parameters, if the biological model is based on more than one model parameters. Thus, the subsequent experimental stimulus may be determined such that the significant environmental condition set by the subsequent experimental stimulus corresponds to a function of previous environmental conditions, in particular to a function of the first environmental condition and of the second environmental condition.

Once the subsequent experimental stimulus is determined as described, the subsequent experimental stimulus may be applied to at least one of the one or more biological probes by the probe manipulation device, and the method may be set forth. Thus, the stimulus trajectory may be extended by a subsequent experimental stimulus to be applied to the one or more biological probes after the experimental stimuli of the initial stimulus trajectory have already been applied to the one or more biological probes, wherein the subsequent experimental stimulus is chosen to be at a region of extremal, e.g. maximal or minimal, relative variation of the cellular parameter. For example, the stimulus trajectory may be a sequence of drug concentrations and the cellular parameter determined by the analysis unit may be a cell death rate of the one or more biological probes. Then, the subsequent experimental stimulus may be a drug concentration between a first and a second drug concentration, between which a maximal variation of death cell rate has been previously determined upon applying a previous stimulus trajectory, for example using the biological model.

By determining a subsequent experimental stimulus this way, the one or more biological probes can be made to evolve to experimental conditions providing higher significance for accurately determining the one or more model parameters of the biological model. Thus, highly informative data points are thereby selected, such that the process of parameter estimation for the biological model is efficiently optimised. Thereby, environmental conditions of the one or more biological probes, at which data points are highly informative about model parameters of interest, can be selected and applied without requiring the intervention of a human operator in real time. An example of a related method for acquiring experimental data is provided below in Example 2.

According to some embodiments, the method of the present invention, in particular the method according to any of the embodiments described herein, may be simultaneously carried out in and/or by a plurality of live-cell imaging systems. The live-cell imaging systems may be located remote from each other and connected to a central control system, such as a central processing module, for example via an internet connection. Thus, a corresponding plurality of imaging devices, and probe manipulation devices may be used for obtaining data points in the form of measurement values of the same or different cellular parameters and for respectively applying the same or different experimental stimuli. For example, if the regulatory task defines a stimulus trajectory and all imaging systems measure the same cellular parameter, more than one experimental stimulus of the stimulus trajectory may be applied at once by different live-cell imaging systems such that a stimulus trajectory is distributed over a larger number of imaging devices and biological probes and can hence be completed in a shorter time, as compared to a situation in which only one live-cell imaging system is used. Different live-cell imaging systems may apply equal or different stimulus trajectories.

In some embodiments, different regulatory tasks defining different stimulus trajectories may be applied to different biological probes, at the same or by different live-cell imaging systems. The different stimulus trajectories may be applied sequentially by one probe manipulation device and/or simultaneously by a plurality of probe manipulation devices. For example, different biological probes may be exposed to a stimulus trajectory defined as a function, for example a time-dependent function, and determined by one or more stimulus parameters of said function. Differently varying, for example time varying said function, experimental stimuli corresponding to different values of the one or more stimulus parameters may be applied to different biological probes in parallel, i.e. simultaneously. The different experimental stimuli may be sequentially applied by the probe manipulation device of one live-cell imaging system. However, each of the different experimental stimuli may additionally or alternatively be applied by a corresponding probe manipulation device, wherein each probe manipulation device may be part of a corresponding live-cell imaging system, such that the different experimental stimuli can be simultaneously applied.

Additionally or alternatively, different stimulus trajectories may be applied to different biological probes, for example by the probe manipulation device of a live-cell imaging system, by applying a stimulus trajectory defined as a function determined by one or more stimulus parameters, wherein different values of the one or more stimulus parameters are chosen for the different biological probes.

In preferred embodiments of the invention, the different stimulus trajectories may be determined or defined by different values of at least one stimulus parameter and the method may further comprise determining an extremal value of the at least one stimulus parameter, wherein the extremal value of the at least one stimulus parameter corresponds to an experimental stimulus of the different stimulus trajectories for which an extremal measurement value is determined. The measurement values may be determined based on a biological model as previously explained.

The different stimulus trajectories applied to the different biological probes may trigger different evolutions and values of the cellular parameter. As a consequence, the cellular parameter may take different values for the different biological probes along the corresponding stimulus trajectory. The extremal value of the at least one stimulus parameter may be defined as the value at which an extremal, e.g. maximal or minimal, measurement value of the cellular parameter is determined. "Extreme" may refer in this case to extreme out, e.g., maximal or minimal, with respect to the experimental stimuli of the different stimulus trajectories. For example, a maximum value of a stimulus parameter X may correspond to an experimental stimulus U(X) applied to one of the biological probes, for which a measurement value is determined, which is smaller (in the case of being minimal) or greater (in the case of maximal) than any other measurement values for the different stimulus trajectories.

For example, the measured cellular parameter may be a cell death rate and the stimulus trajectories may correspond to sequences of concentrations of a reactant to which the one or more biological probes are exposed, wherein each concentration may be defined as a time-dependent function dependent on a constant related to an injection rate of the reactant (the different stimulus trajectories may be respectively defined by different injection rates). Once it is considered that all stimulus trajectories have been completed for the different biological probes for the respective values of the injection rate, for example upon reaching a predefined time limit, the extremal value can be determined as the "extremal" injection rate at which a maximal or minimal cell death rate is observed taking into account all biological probes or a subset thereof.

First and second reference values of the stimulus parameter may then be determined such that the first reference value is smaller than the extremal value and the second reference value is greater than the extremal value, i.e. such that the extremal value is between the first reference value and the second reference value. For example, the first and second reference values may correspond to respective reference injection rates, being respectively smaller and greater than the aforesaid extremal injection rate for which the maximal (or minimal) cell death rate is observed.

The different stimulus trajectories may then be replaced updated by respective updated stimulus trajectories for the different biological probes and applied thereto by the probe manipulation device or the respective probe manipulation devices, wherein the updated stimulus trajectories are determined by different values of the at least one stimulus parameter ranging between the first and second reference values. Thereby, a new set of stimulus trajectories is defined for values of the at least one stimulus parameter concentrated around the extremal value that resulted in an extremal measurement value.

By repeating the process of determining updated stimulus trajectories based on different values of the at least one stimulus parameter as previously explained, for example for a predefined number of iterations or until a convergence criterion for the stimulus parameter is fulfilled, the value of the cellular parameter can be maximised or minimised via the experimental stimuli, even in the absence of a priori knowledge about the environmental conditions favouring such extremal state of the cellular parameter. The aforesaid convergence criterion for the experimental stimulus may be defined as a threshold difference between the first and second reference values. An example of a related method for acquiring experimental data is provided below in Example 3.

A further aspect of the invention refers to a live-cell imaging system for acquiring experimental data of one or more biological probes. The live-cell imaging system comprises an imaging device for obtaining at least one optical measurement of the one or more biological probes. The live-cell imaging further comprises a probe manipulation device for applying at least one experimental stimulus to the one or more biological probes. The at least one experimental stimulus sets an environmental condition of the biological probe to which the experimental stimulus is applied. In other words, the at least one experimental stimulus influences and environmental condition of the corresponding probe to which it is applied. The live-cell imaging system further comprises a control unit configured for controlling the operation of the imaging device and the probe manipulation device based on control instructions received over a functional connection. The control unit may hence be operatively connected between the functional connection on one side and the imaging device and the probe manipulation device on the other side. The functional connection may comprise a connection port or connection terminal, or any I/O-means or connector allowing for the exchange of information, including input and/or output information, between the control unit on the one side and an exterior of the live-cell imaging system on the other side. The functional connection may in particular be a wired connection, such as an Ethernet connection or the like, or a wireless connection, such as a WLAN, Bluetooth or radio connection or the like.

The control unit may be hardware-based, for example in the form of a processor located in a proximity of the imaging device and/or the probe manipulation device, or as a remote processor connected to the imaging device over a wired or wireless connection, for example via the internet. However, the control unit may also be software-based and be installed on a processor, which may also be a remote processor or a processor located in proximity of the imaging device and/or the probe manipulation device.

The control unit may be configured for outputting over the functional connection information comprising the at least one optical measurement obtained by the imaging device and/or information related thereto. Said information may in particular comprise at least one measurement value obtained from the at least one optical measurement. For example, the live-cell imaging system may comprise the imaging device, the probe manipulation device, the control unit and a processing module configured for determining, based on the at least one optical measurement obtained by the imaging device, at least one measurement value of the cellular parameter of the one or more biological probes. The aforesaid information may then comprise the at least one measurement value and the control unit may be configured for outputting over the functional connection the at least one measurement value determined by the processing module. The outputted at least one measurement value may be transmitted to an external device.

In other examples, the live-cell imaging system may comprise the imaging device, the probe manipulation device, and the control unit but no integrated or local processing module. The information outputted by the control unit may then comprise the at least one optical measurement and the control unit may be configured for outputting, over the functional connection, the at least one optical measurement obtained by the imaging device, for example to an external processing module, to be described in more detail below. Such an external processing module may be configured for implementing the previously described live-cell imaging method of the invention, in particular for determining, based on the at least one optical measurement received over the functional connection, at least one measurement value of the cellular parameter of the one or more biological probes, and for processing the at least one measurement value for generating control instructions to be sent to the live-cell imaging system over the functional connection. The control instructions may in particular comprise control instructions to generate and/or apply at least one experimental stimulus determined by the processing module for being applied by the probe manipulation device according to a regulatory task.

The control unit is further configured for controlling the probe manipulation device to apply the at least one experimental stimulus to the one or more biological probes according to the control instructions received over the functional connection. The control unit is operatively connected to the probe manipulation device. The control instruction may be a "raw instruction" corresponding to a target value of the cellular parameter or a related quantity, to be translated by the probe manipulation device into a corresponding experimental stimulus. The probe manipulation device may comprise or be connectable or connected for this purpose to a corresponding implementation software tool or processor configured for deriving an experimental stimulus required for implementing such a control instruction. The control instruction may however also be a "pre-processed instruction" taking into account the configuration of the probe manipulation device and corresponding to an experimental stimulus to be applied by the probe manipulation device, for example a light intensity or a flow of a reactant. The control unit may comprise means for generating or receiving such a "pre-processed" control instruction.

The imaging device may comprise an optical device, in particular one or more of a microscope, a digital camera, a CCD, one or more mirrors, one or more deflectors, and/or one or more focusing lenses.

According to some embodiments, the imaging device or the optical device may be movable for scanning the one or more biological probes. For example, the imaging device may be movable in at least one or two dimensions, or in three dimensions, for scanning the one or more biological probes. If the imaging device comprises an optical device, for example a microscope, the optical device itself may be movable, while other components of the imaging device may or not be movable. For instance, if the one or more biological probes are arranged on a transparent plate such as to be optically accessible by the imaging device, all biological probes may be arranged on a same plane and it may be sufficient for the imaging device to be movable in the two directions defined by said plane in order to be able to scan all probes. If the one or more biological probes are arranged on a plurality of plates that are arranged over each other, at different heights, the imaging device or the optical device may further be movable in a preferred direction, not parallel to any of the first or second directions, such as to optically access biological probes arranged on different plates.

The control unit may further be configured for controlling a displacement of the imaging device, for example based on displacement instructions received over the functional connection. For each direction of movement of the imaging device, the imaging device may comprise a guide structure and a motor unit, for example a stepper motor, configured for implementing a movement instruction received from the control unit into a movement of the imaging device in the corresponding direction over the corresponding guide structure. It should be understood that an optical device may be movable for scanning the biological probes while the motor units and the guide structures need not be movable, at least not to the same extent as the optical device.

The live-cell imaging system may comprise a housing enclosing at least some of the remaining components of the imaging device, in particular enclosing one or more of the imaging device, the control unit, the probe manipulation device and the control unit. One or more of these components may however be arranged outside of the housing. For example, the imaging device, in particular with corresponding motor units, and optionally the control unit may be arranged within the housing, whereas the probe manipulation device may be arranged outside of the housing. The control unit may also be arranged outside of the housing.

The housing may be separable from the rest of the live-cell imaging system, for example for undergoing a sterilisation process, for example an autoclavation process.

In some embodiments, the housing may comprise a cover plate, a bottom plate and a least a lateral wall extending between the cover plate and the bottom plate. For example, if the housing has a cylindrical form, one lateral wall may be provided, and if the housing has a hexahedral from, for example an approximately cubical or rectangular parallepipedal form, four lateral walls may be provided. The cover plate may be at least partly transparent and may be configured for supporting the one or more biological probes and/or one or more probe carriers, for example Petri dishes, well plates, multi-well plates or the like, containing the one or more biological probes. The one or more probe carriers may be at least in part of a transparent material. Since the cover plate is at least in part transparent, it allows optical access of the one or more biological probes by the imaging device through the cover plate. In some embodiments, the housing may comprise a bottom plate and/or one or more lateral walls comprising a metal or another thermal conducting material, which may facilitate thermal equilibration between the interior and an exterior of the housing.

In preferred embodiments, the live-cell imaging system may comprise a reflective element, for example a mirror or a mirror plate, for directing illumination light to and/or through the one or more biological probes. The live-cell imaging system may further comprise an illumination light source for generating light for illuminating the one or more biological probes for obtaining the at least one optical measurement by the imaging device. The illumination light may comprise an LED light source.

In some embodiments, the reflective element may comprise a mirror plate arranged over the cover plate, in particular the transparent cover plate, of the housing, for example parallel to the cover plate. The illumination light source may be arranged within the housing and/or within the imaging device, for example within the objective of a microscope or camera of the imaging device. The reflective element may then be arranged such as to reflect light generated by the illumination light source back through the one or more biological probes arranged on the cover plate, such that the illumination light reflected by the reflective element can be efficiently used for obtaining the at least one optical measurement of the biological probes by means of this compact configuration.

In some preferred embodiments, the reflective element may be movable with respect to the cover plate such as to allow access to the cover plate, for example for arranging or removing biological probes on the cover plate. For example, the reflective element, in some examples the mirror plate, may be pivotably connected to the housing and be pivotable between a closed position and an open position. In the closed position, the reflective element may be arranged substantially parallel to the cover plate and be configured for reflecting illumination light through at least some of the one or more biological probes, for example towards the interior of the housing and/or towards the imaging device. In the open position, the reflective element may be displaced from the closed position so as to allow access to the cover plate. Biological probes arranged on the cover plate may then be arranged between the cover plate and the reflective element, for example the mirror plate, when the reflective element is in the closed position, while said biological probes may be exposed when the reflective element is in the open position.

According to some embodiments, the probe manipulation device may comprise a perfusion device configured for perfusing the one or more biological probes with an experimental fluid, i.e. to let the experimental fluid flow in contact with the one or more biological probes, thereby influencing an environmental condition of the one or more biological probes. The perfusion device may comprise a fluid pump, fluid conduits and/or fluid connectors for controlling and guiding the flow of the experimental fluid.

In some embodiments, the probe manipulation device may comprise a light source, preferably an LED, for emitting experimental light upon the one or more biological probes. This allows the probe manipulation device to set or influence an environmental condition of the one or more biological probes by means of the light to which the one or more biological probes are exposed, for instance by correspondingly setting the light intensity and/or light wavelength.

A further aspect of the invention refers to a processing module connectable or connected to the functional connection of the live-cell imaging system of any of the previously described examples. In some embodiments, the processing module may be understood as a part of a live-cell imaging system according to the invention, in particular when it is connected to the functional connection. The processing module may be configured for implementing the live-cell imaging method according to any of the previously discussed examples or embodiments of the method of the invention as described herein.

The processing module may be configured for receiving, over the functional connection, information comprising at least one optical measurement or related thereto, in particular the information outputted by the control unit of the live-cell imaging system.

The processing module may further be configured for determining, based on the aforesaid information, for example based on the at least one optical measurement, at least one measurement value of a cellular parameter of the one or more biological probes. For this purpose, the processing module may comprise, in some preferred embodiments, an artificial intelligence algorithm, in particular a neural network algorithm, for example a convolutional neural network algorithm, trained for determining the at least one measurement value from the at least one optical measurement. The artificial intelligence algorithm may be configured for classifying, counting and/or identifying cells in the one or more biological probes with respect to the cellular parameter based on the obtained optical measurement. The algorithm may for example allow the analysis unit to classify cells in the one or more biological probes as living cells or dead cells and/or to count the numbers thereof based on an image of the respective biological probe.

The processing module may further be configured for determining whether at least one measurement value received over the functional connection or at least one measurement value determined from at least one optical measurement received over the functional connection satisfies a convergence criterion of a regulatory task. The convergence criterion and the regulatory task may respectively correspond to any of the convergence criteria and regulatory tasks that have been previously explained with respect to the method according to the present invention.

The processing module is further configured for, if the convergence criterion is not satisfied, determining at least one experimental stimulus. The at least one experimental stimulus is determined to set an environmental condition of the biological probe which the experimental stimulus is applied so as to improve fulfilment of the convergence criterion, i.e. in such a manner that the convergence criterion of the regulatory task is fulfilled or is better fulfilled as compared to the situation before applying the determined at least one experimental stimulus, for example based on a biological model for estimating values of the cellular parameter as previously described.

Notably, determining, by the processing module, such an experimental stimulus improving fulfilment of the convergence criterion of the regulatory task may be achieved in different manners, in particular in manners described above, which are all covered by the scope of the present invention. For example, if the regulatory task defines a target value of the cellular parameter of the biological probe, the experimental stimulus may be determined such that it causes the corresponding biological probe to which the experimental stimulus is applied to evolve towards a situation in which the cellular parameter takes the aforesaid target value, or at least takes a value of the cellular parameter closer to the aforesaid target value as compared to the situation before applying be at the determined experimental stimulus.

The same applies to other examples in which the regulatory task defines a target value of a function of the cellular parameter, such as a derivative thereof (e.g. such that the cell division rate is maximal or minimal). In other examples, the regulatory task may define a target value of the cellular parameter such that the cellular parameter follows a predefined parameter trajectory or achieves a desired extremal state, such as a predefined time evolution, and the experimental stimulus may be determined such that it causes the corresponding biological probe to which the experimental stimulus is applied to evolve according to said predefined parameter trajectory or time evolution, for example based on a biological model for estimating values of the cellular parameter as previously described. In other examples, the regulatory task may define a target environmental condition of the one or more biological probes to which the experimental stimulus is applied, and the experimental stimulus may be determined by the processing module such that it causes the corresponding environmental condition to achieve or at least evolve to said target environmental condition, for example based on a biological model for estimating values of the cellular parameter as previously described.

The processing module is further configured for sending control instructions through the functional connection to the live-cell imaging system for controlling the probe manipulation device, in particular via the control unit of the live-cell imaging system, to apply the at least one experimental stimulus determined by the processing module as explained above to the one or more biological probes.

The processing module may in particular be configured for controlling the live-cell imaging system so as to implement the method according to any of the examples or embodiments previously described.

A further aspect of the present invention refers to a digital storage device comprising executable code which, when executed by a processor, configures the processor for operating as a processing module according to any of the aforementioned examples or embodiments.

BRIEF DESCRIPTION OF THE FIGURES

Fig. 1    schematically illustrates a live-cell imaging systems according to embodiments of the invention.

Fig. 2    schematically illustrates an imaging device of a live-cell imaging system according to embodiments of the invention. Fig. 2a is a side view and Fig. 2b a top view of the imaging device.

Fig. 3    schematically illustrates components of a live-cell imaging system according to embodiments of the present invention.

Fig. 4    schematically illustrates a method according to embodiments of the invention.

Fig. 5    schematically illustrates a method according to embodiments of the invention.

Fig. 6    schematically illustrates a method according to embodiments of the invention.

Fig. 7    schematically illustrates a method according to embodiments of the invention.

Fig. 8    schematically illustrates a system of live-cell imaging systems according to embodiments of the invention.

Fig. 9    schematically illustrates a method according to embodiments of the invention.

DESCRIPTION OF PREFERRED EMBODIMENTS OF THE INVENTION

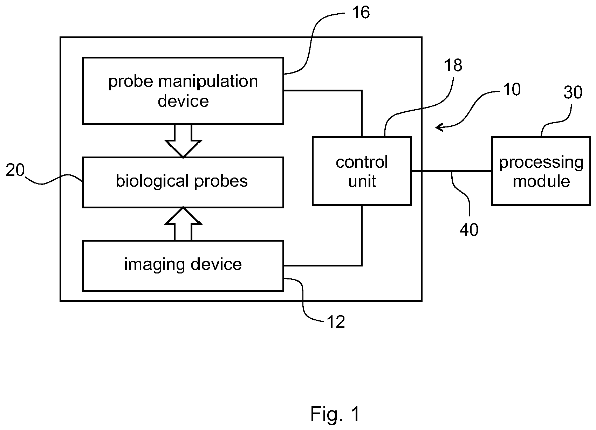

Fig. 1 schematically illustrates a live-cell imaging system 10 according to embodiments of the present invention. The live-cell imaging system 10 comprises an imaging device 12 configured for obtaining at least one optical measurement of a plurality of biological probes 20. The arrow between the imaging device 12 and the biological probes 20 in Fig. 1 signals an optical access between the imaging device 12 and the biological probes 20 that allows the imaging device 12 to obtain the at least one optical measurement. In particular, the imaging device 12 comprises, in the exemplary embodiment under consideration, a microscope configured for obtaining the at least one optical measurement of the biological probes 20. The biological probes 20 are probes containing cells in an environment, wherein the environment is provided by a solution, and wherein the cells and their environment are contained in a transparent well plate.

The live-cell imaging system 10 further comprises a probe manipulation device 16 configured for applying at least one experimental stimulus to the one or more biological probes 20. The arrow between the probe manipulation device 16 and the biological probes 20 in Fig. 1 signals an interaction capability of the probe manipulation device 16 upon the biological probes 20 for setting an environmental condition thereof. The probe manipulation device 16 can for example comprise a perfusion device configured for perfusing the biological probes 20 with an experimental fluid, wherein the experimental fluid comprises a bioactive agent that may stimulate death cell, thereby influencing an environmental condition thereof by modifying the properties of the environment of the biological probes, i.e. the solution containing the biological probes. The probe manipulation device can then be configured to control a concentration of the bioactive agent in the experimental fluid. A higher concentration of the bioactive agent induces a higher apoptosis rate of cells in the biological probes 20, and a lower concentration of the bioactive agent induces a lower apoptosis rate.

Additionally or alternatively, the probe manipulation device 16 can comprise a light source, such as an LED (not shown in the figure), for emitting experimental light on the one or more biological probes 20, thereby influencing an environmental condition thereof by means of the emitted light.

The live-cell imaging system 10 further comprises a control unit 18 that is operatively connected to the imaging device 12 and to the probe manipulation device 16. The control unit 18, which in the exemplary embodiment shown is a software-based control unit 18 supported on an internal processing unit of the live-cell imaging system 10, is configured for controlling the operation of the imaging device 12 and the probe manipulation device 16 based on control instructions received over a functional connection 40, over which the live-cell imaging system 10, and in particular the control unit 18, is connectable to external devices. In the embodiment shown, the functional connection 40 is a wired connection, for instance an Ethernet connection for inputting and outputting data over the internet. However, in other embodiments, the functional connection 40 may be another type of wired connection or a wireless connection.

By means of the functional connection 40, the control unit 18 is connected to an external processing module 30. The control unit 18 is configured for receiving control instructions from the external processing module 30, and for controlling the operation of the imaging device 12 and the probe manipulation device 16 based on such control instructions. For example, the probe manipulation device 16 sets the concentration of the bioactive agent in the experimental fluid as controlled by the control unit 18 and controls a position of the imaging device 12 with respect to the biological probes 24 obtaining optical measurements according to a measuring routine obtained through the functional connection 40.

Further, the control unit 18 is configured for outputting to the processing module 30, over the functional connection 40, the optical measurements of the biological probes 20 obtained by the imaging device 12. The processing module 30 is configured for analysing the optical measurements obtained by the imaging device 12 received over the functional connection 40 and for determining at least one measurement value of a cellular parameter of the one or more biological probes 20. The processing module 30 comprises a neural network algorithm trained for classifying cells of the biological probes 20 based on corresponding optical measurements with respect to a chosen cellular parameter. For instance, the algorithm may be configured and trained for determining a number of living cells and/or a number of dead cells in a biological probe 20 from an optical measurement thereof, for example by means of image segmentation.

In the exemplary embodiment illustrated in Fig. 1, the processing module 30 is an external processing module connected to the live-cell imaging system 10 over the functional connection 40. However, in other embodiments of the invention the functions of the processing module or a part thereof may be performed by an internal processing unit. The processing module 30 may be a hardware-based module or a software-based module, for example in the form of software code loaded on a processor. The processing module 30 is operatively connected, via the functional connection 40, to the control unit 18.

The processing module 30 is further configured for determining, if the convergence criterion is not satisfied, at least one experimental stimulus for setting environmental condition of the biological probes 20 by means of the probe manipulation device 16. The processing module is configured for determining the at least one experimental stimulus such that the at least one experimental stimulus, upon being applied by the probe manipulation device 16 to the one or more biological probes 20, set corresponding environmental conditions of the biological probes 20 such that fulfilment of the convergence criterion is improved.

The processing module 30 is further configured for sending control instructions to the live-cell imaging system, in particular to the control unit 18, via the functional connection 40, for controlling the probe manipulation device 16 to apply the at least one experimental stimulus determined by the processing module 30 to the one or more biological probes 30. This can be achieved by different configurations that may allow the processing module 30 to control a live-cell imaging system so as to implement a live-cell imaging method according to embodiments of the present invention. Examples of some of these configurations are discussed below.

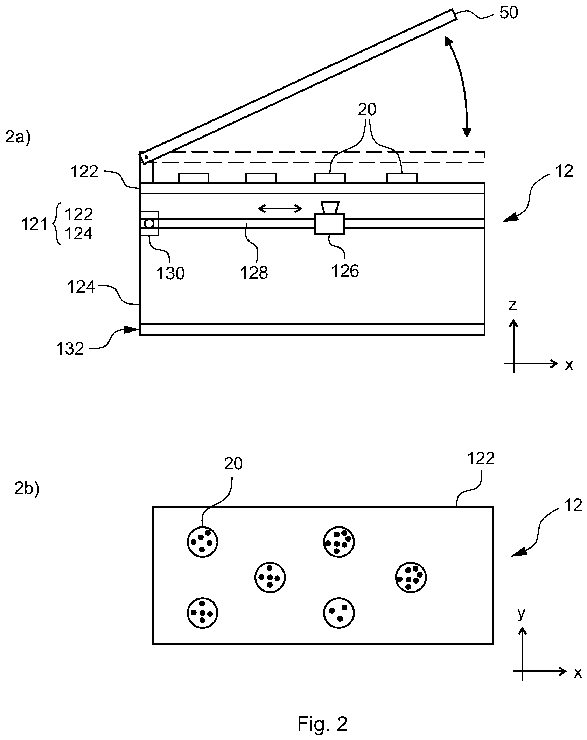

Fig. 2 shows a schematic illustration of the imaging device 12 of a live-cell imaging system according to embodiments of the invention. The imaging device 12 comprises a microscope 126 configured for obtaining the optical measurements of the biological probes 20. The imaging device 12 comprises a housing 121 that encloses other components of the live-cell imaging system (not shown in the figure). In the case of the live-cell imaging system 10 shown in Fig. 1, the housing 121 can for example enclose the imaging device 12 and the control unit 18.

Fig. 2a illustrates a side view of the imaging device 12. The housing 121 comprises a transparent cover plate 122. The one or more biological probes 20 are respectively contained in transparent well plates and are arranged on the cover plate 122, as illustrated in Fig. 2b, which represents a top view of the imaging device 12. Although six biological probes are schematically illustrated in Fig. 2b, larger numbers of probes may be arranged on the live-cell imaging device 10 in order to obtain optical measurements from them.

The microscope 126 is movable for scanning the one or more biological probes 20. For this purpose, the imaging device 12 comprises a first guide structure 128 for guiding the movement of the microscope 126 in the x direction and a first stepper motor 130 for driving the movement of the microscope 126 along the first guide structure 128, i.e. in the x direction. Although it is not shown in Fig. 2, the imaging device 12 further comprises a second guiding structure for guiding the movement of the microscope 126 in the y direction and a corresponding second stepper motor for driving the movement of the microscope 126 along the second guide structure, i.e. in the y direction. Means for adjusting the position of the microscope 126 in the z direction can also be provided.

The control unit 18 is further configured for controlling a movement and corresponding positioning of the optical device by correspondingly controlling the settings of the stepper motors or of the corresponding motor units for scanning the one or more biological probes 20 according to a measuring routine, which can be stored in the control unit 18 or be inputted to the control unit 18 by the processing module 30. The measuring routine specifies a sequence of measuring events, i.e. of measuring positions corresponding to one of the probes and respective measuring times.

When optical measurements are to be obtained from a particular biological probe 20, the position of the microscope 126 is adjusted such that the microscope 126 is located directly below said particular biological probe 20 and can optically access the probe through the transparent cover plate 122 and through the corresponding transparent well plate, dish, well or the like for obtaining the optical measurements. The coordinates defining the position of each biological probe on the plate 122 can be stored in the control unit 18 or in an external processing module 30. The microscope 126 can then be moved so as to scan the biological probes 20 in order to obtain optical measurements of the biological probes 20, for example based on control instructions received from the processing module 30 over the functional connection 40 corresponding to the positions of the biological probes 20 on the cover plate 122. In other examples, the control unit 18 or the processing module 30 can be configured for identifying non-pre-stored positions of the biological probes 20.

In the embodiment shown in Fig. 2, the housing 121 has an hexahedral shape. Thus, the cover plate 122 has a rectangular shape and the housing 121 has four lateral walls 124 that extend between the cover plate 122 and a bottom plate of the housing 121. However, in other embodiments, the housing may have a different shape and a corresponding different number of lateral walls.

In the embodiment shown in Fig. 2, as can be seen in the side view of Fig. 2a, the microscope 126 comprises an LED source for generating illumination light. When the microscope 126 is obtaining an optical measurement of a given one of the biological probes 20, said given biological probe can be illuminated by the illumination light generated by the LED source through the transparent cover plate 122.

The live-cell imaging system 10 illustrated in Fig. 2 further comprises a mirror plate 50 that is pivotally connected to the housing 121 and can pivot between a closed position, which is indicated in Fig. 2a with dashed lines, and an open position, illustrated in Fig. 2a with solid lines. When the mirror plate 50 is in the closed position, the mirror plate 50 reflects back the illumination light generated by the LED source towards the LED source, i.e. towards the microscope 126, such that the microscope 126 can obtain one or more optical measurements of the rear-illuminated biological probe 20. In the closed position, the mirror plate 50 is arranged parallel to the transparent cover plate 122 and the biological probes 20 arranged on the cover plate 122 are arranged between the cover plate 122 and the mirror plate 50.

The mirror plate 50 can be pivoted to the open position, shown in Fig. 2a with solid lines, such that the mirror plate 50 is tilted with respect to the cover plate 122 and allows access to the cover plate 122 for arranging biological probes 20 on the cover plate 122 and for removing biological probes 20 from the cover plate 122.

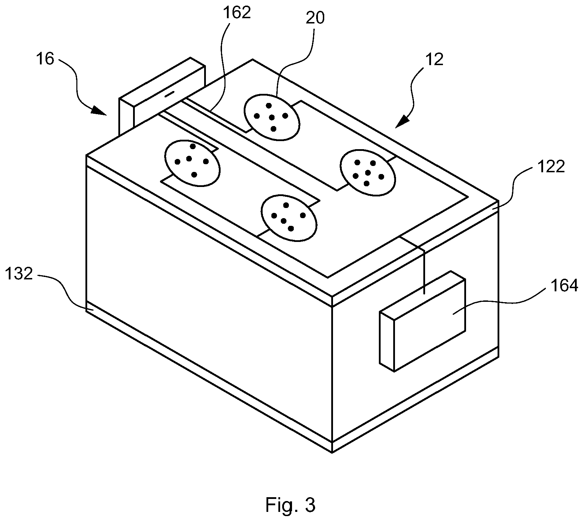

Fig. 3 schematically illustrates a perspective view of the housing enclosing the components of a live-cell imaging system 10 according to embodiments of the invention. A number of well plates containing the biological probes 20 are arranged on the cover plate 122 of the housing. The hexahedral housing further comprises a metallic bottom plate 132. The probe manipulation device 16 is configured for perfusing the biological probes 20 with an experimental fluid via experimental fluid conduits 162 that connect each of the biological probes 20 between the probe manipulation device 16 and an experimental fluid output 164 that collects or outputs the experimental fluid after it has flown in contact with the biological probes 20. The probe manipulation device 16 comprises a fluid pump for driving a flow of the experimental fluid.

Fig. 4 is a flow diagram schematically illustrating a live-cell imaging method 200 according to an embodiment of the invention which may be implemented by a live-cell imaging system, for example by the live-cell imaging system 10 of Fig. 1 under the control of the processing module 30.

According to the method 200, at least one optical measurement of the biological probes 20 is obtained by the imaging device 12 at operation 202. The at least one optical measurement can correspond to digital image data of the one or more biological probes 20. For instance, each optical measurement can correspond to digital image data of one corresponding biological probe 20 at a given time. The control unit 18 controls the operation of the imaging device 12 for obtaining the at least one optical measurements, for example by determining the settings of the optical device of the imaging device 12 for the optical measurement, such as focusing settings or image size and definition, and/or by determining a positioning or sequence of positionings of the optical device with respect to the one or more biological probes 20. The optical measurements obtained are then received by the control unit 18 and forwarded to the processing module 30 via the functional connection 40. The optical measurements can correspond to one of the biological probes 20 or to different biological probes 20, in which case the information received by the control unit 18 from the imaging device 12 can further comprise, for each optical measurement, information about the corresponding biological probe, for example spatial coordinates or an identification label obtained during the measurement by the imaging device 12.

According to the method 200, at 204, the processing module 30 analyses the at least one optical measurement obtained by the imaging device 12 for determining at least one measurement value of a cellular parameter of the one or more biological probes 20. The cellular parameter can for example be an apoptosis rate based on counts of living and/or dead cells extracted from timely distributed optical measurements of the same biological probe, the measurement value then corresponding to the value of the apoptosis rate in each case. For this purpose, the processing module 30 of the embodiment considered comprises a convolutional neural network algorithm that has been trained using a large number of optical measurements for identifying cells as living and/or dead cells from an image of the corresponding biological probe obtained as an optical measurement. From the values of the measurement value "number of apoptotic cells", a cellular parameter "apoptosis rate" or a cellular parameter "drug concentration at half-maximal apoptosis rate" can be estimated using a biological model, as will exemplarily be shown below.

The processing module 30, at 206, determines whether the at least one measurement value satisfies a convergence criterion of a regulatory task. In the embodiment under consideration, an example of which is provided in detail below as Example 1, the regulatory task may define a target apoptosis rate to be achieved and held, for example an apoptosis rate of 0.1/h.

If the result of operation 206 is positive, i.e. if the processing module 30 determines that the convergence criterion is satisfied, this means that the apoptosis rate has a value corresponding to the target apoptosis rate and the method is terminated, at 208, as illustrated in Fig. 4. In other examples, after positive result in 206, the method 200 may go back to operation 202, for example for maintaining fulfilment of the regulatory task by keeping the apoptosis rate at a constant value corresponding to the target apoptosis rate.

If the result of operation 206 is negative, i.e. if the processing module 30 determines that the convergence criterion is not satisfied, this means that the apoptosis rate does not (yet) have a value corresponding to the target apoptosis rate. The method 200 then continues, at 210, with the probe manipulation device 16 of the live-cell imaging system 10 applying to the one or more biological probes 20 an experimental stimulus, for example a drug concentration determined by the processing module 30, such that the apoptosis rate approaches or achieves the target apoptosis rate. If the regulatory task is not fulfilled because the determined apoptosis rate is below the target apoptosis rate, the processing module 30 defines an experimental stimulus corresponding to an increase in the concentration of the bioactive agent in the experimental fluid and correspondingly instructs the probe manipulation device 16, via the control unit 18, to set the concentration accordingly. As a result, the apoptosis rate of the biological probes, to which the experimental stimulus is applied, increases.

Conversely, if the determined apoptosis rate is above the target apoptosis rate, the processing module may determine and transmit to the control unit 18 an experimental stimulus corresponding to a reduction in the concentration of the bioactive agent in the experimental fluid with which the biological probes 20 are being perfused. As a result, the apoptosis rate of the biological probes, to which the experimental stimulus is applied, decreases.

The method then goes back to operation 202 and reiterates until the regulatory task is fulfilled, or reiterates in order to keep fulfilment of the regulatory task.

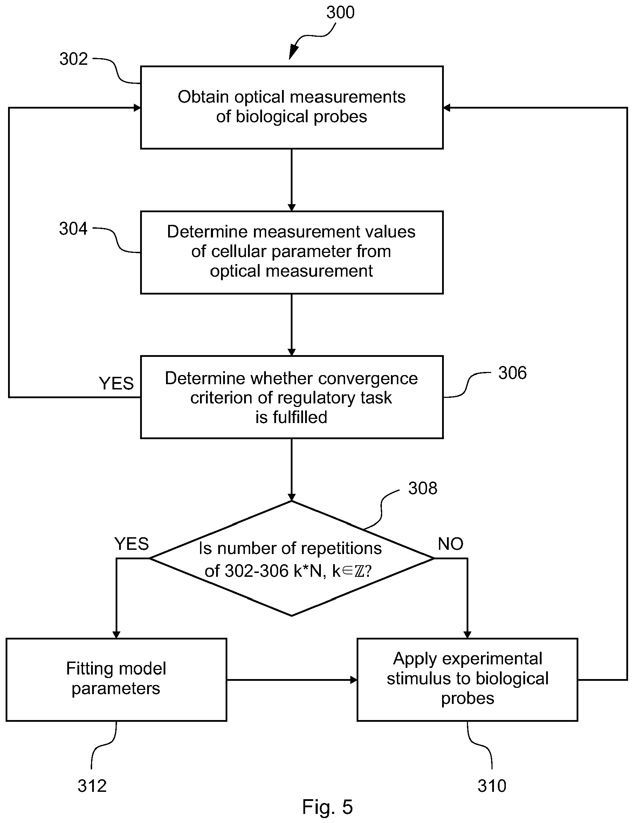

Fig. 5 is a flow diagram schematically illustrating a live-cell imaging method 300 according to an embodiment of the invention which may be implemented by a live-cell imaging system, for example by the live-cell imaging system 10 of Fig. 1 under the control of the processing module 30. The method 300 is a variation of method 200 illustrated in Fig. 4. A detailed explanation of operations 302 to 306 and 310 of method 300, which correspond, respectively, to method operations 202 to 206 and 210 of method 200 previously explained with reference to Fig. 4 is omitted for brevity. In the method illustrated in Fig. 5, contrary to the method illustrated in Fig. 4, a positive result of the condition evaluation in operation 306, i.e. if the processing module 30 determines that the regulatory task is being fulfilled, the method 300 is continued by going back to operation 302.

According to the method 300, the processing module 30 is configured for recording a sequence of N measurement values and associated environmental conditions for each of the biological probes 20 corresponding to N cyclic repetitions of operations 302 and 304. In the example illustrated in Fig. 5, the cyclic repetitions also include method operation 306, but this needs not be the case in other examples.

The associated environmental conditions correspond to the environmental conditions of a biological probe when the optical measurement is obtained and may be estimated from the optical measurement and/or from an experimental stimulus applied to the biological probe, or directly measured by other means such as sensors and the like. For example, when the at least one experimental stimulus corresponds to a light intensity, for instance provided by an LED light source, to which the one or more biological probes are exposed, the imaging device may comprise a light intensity detector for detecting the light intensity applied to the biological probe from which the optical measurement is being obtained. Additionally or alternatively, the probe manipulation device may be calibrated such that an environmental condition can be directly obtained from the experimental stimulus applied to the biological probe from which the optical measurement is being obtained.

N may for example be 10 or 100. If the processing module 30 determines at 306 that the regulatory task is not fulfilled, the processing module 30 evaluates, at 302, whether a number of consecutive cyclic repetitions of operations 302 to 306 corresponds to N or to a multiple thereof, i.e. to $k \cdot N$ with k being an integer ($k \in Z$). If the number of cyclic repetitions of operations 302 to 306 does not correspond to $k \cdot N$, the method 300 continues with operation 310, which is analogous to operation 210 described above for the method 200 of Fig. 4, i.e. with the probe manipulation device 16 of the live-cell imaging system 10 applying to the one or more biological probes 20 an experimental stimulus, for example a drug concentration determined by the processing module 30, such that the apoptosis rate approaches or achieves a target apoptosis rate, as previously explained with reference to method 200 of Fig. 4.

If the processing module 30 determines at 308 that the number of cyclic repetitions of operations 302 to 306 corresponds to N or a multiple thereof, method 300 proceeds to operation 312, in which the processing module uses the sequence of measurement values or the sequence of measurement values and associated environmental conditions for fitting model parameters of a biological model for estimating values of the cellular parameter of the one or more biological probes as a function of the corresponding environmental condition and of the model parameters. Thus, the model parameters of the biological model are fitted by the processing module 30 every time operations 302 to 306 are cyclically repeated N times. As a result of operation 312, the biological model is now based on a more accurate estimation of the values of the model parameters and hence has an increased accuracy as compared to the biological model based on the previous or initial values of the model parameters, which might have been initially based on guess values. Following operation 312, the method 300 proceeds to operation 310, and then goes back to operation 302.

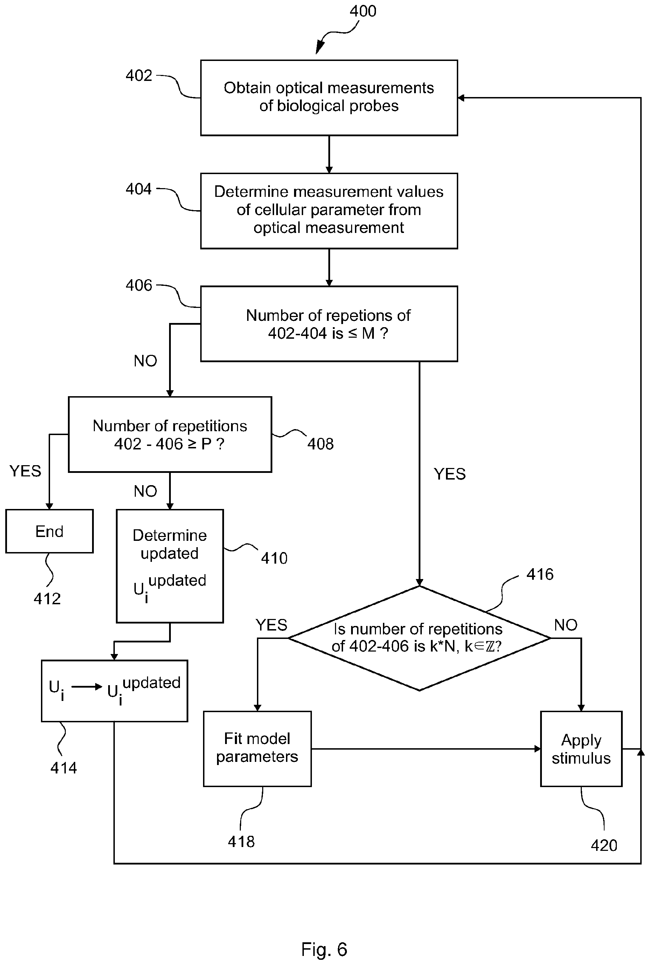

Fig. 6 is a flow diagram schematically illustrating a live-cell imaging method 400 according to an embodiment of the invention which may be implemented by a live-cell imaging system, for example by the live-cell imaging system 10 of Fig. 1 under the control of the processing module 30. The method 400 is a variation of method 200 illustrated in Fig. 5. A detailed explanation of operations 402 and 404 of method 400, which correspond to method operations 302 and 304 of method 300 (and operations 202 and 204 of method 200) previously explained with reference to Fig. 5 (and Fig. 4) is omitted for brevity.

In the exemplary method illustrated in Fig. 6, the cellular parameter being measured or estimated is an apoptosis rate. The processing module 30 comprises a convolutional network algorithm trained for identifying dead cells by means of image segmentation on the basis of image local contrast using images of a biological probe obtained as an optical measurement. The biological probes 20 are contained, in this embodiment, in 6 different well plates that are arranged on the cover plate 122 of the housing 121 (cf. Fig. 2). Each well plate, i.e. each biological probe, contains ca. $10^5$ cells. The control unit 14 is configured for controlling the imaging device 12 such that the imaging device moves to scan the biological probes 20 and obtains optical measurements for each of the biological probes 20 in intervals of 30 seconds, such that a sequence of optical measurements of the number of dead cells is obtained for each biological probe, wherein the measurements of each sequence are respectively spaced by time intervals of 30 seconds. Based on this time-sequence of measurements, the processing module 30 can determine the number of living and dead cells in each biological probe by means of the convolutional network algorithm. The death rate can then be estimated using a biological model from measurement values of the number of death cells and/or the number of living cells at different times.

The probe manipulation device 16 is configured for controlling the concentration of a bioactive agent and for perfusing the biological probes 20 with the experimental fluid via experimental fluid conduits 162 as shown in Fig. 3.

In the case of method 400 illustrated in Fig. 6, the regulatory task defines a target environmental condition of the biological probes 20 in the form of a sequence $U_1,...,U_M$ of M experimental stimuli Ui, i.e. a stimulus trajectory, to be provided by the probe stimulation device 16. The stimulus trajectory corresponds in this case to a sequence of concentrations of a bioactive agent, e.g. a drug, in the experimental fluid to be applied to the biological probes 20.

In the embodiment under consideration, the stimulus trajectory is applied to each of the biological probes 20 in parallel. Thus, with reference to the arrangement illustrated in Fig. 3, the concentrations of the bioactive agent in the experimental fluid with which each of the biological probes 20 is perfused are equal for all (six) biological probes 20 at equal times. In this configuration, six sets of results can be obtained for the same experimental conditions, thereby increasing the statistical significance of the results. However, in other embodiments other configurations are possible; in particular, different biological probes 20 may be sequentially (one probe after the other) or simultaneously (all probes at a time) perfused with different concentrations of the cell death ligand at a given time.

Each time an optical measurement is obtained for one of the biological probes 20, i.e. each time a measurement of the number of the cellular parameter, the measured measurement value and the corresponding concentration of the bioactive agent, i.e. the corresponding value Ui or a value of an environmental condition related thereto, are stored, for example in the processing module 30 or in a storage device connected thereto.

In operation 406, the processing module 30 determines whether a number of repetitions of operations 402 and 404 is smaller or equal (i.e. does not exceed) a number M corresponding to the number of experimental stimuli $U_1,...,U_M$ of the stimulus trajectory. If this is the case, i.e. if the stimulus trajectory $U_1,...,U_M$ has not been completed yet, the method 400 proceeds to operation 416.

In operation 416, the processing module 30 determines, like in operation 308 of method 300 illustrated in Fig. 5, whether the number of repetitions of 202 to 402 of corresponds to N or to a multiple thereof, i.e. to k·N with k being an integer ($k \in Z$).

If the number of cyclic repetitions of operations 402 to 406 does not correspond to k·N, the method 400 continues with operation 420, which is analogous to operations 210 and 310 described above, respectively, for method 200 illustrated in Fig. 4 and for method 300 illustrated in Fig. 5, i.e. with the probe manipulation device 16 of the live-cell imaging system 10 applying to at least one of the one or more biological probes 20 an experimental stimulus, for example a drug concentration (or e.g. a light intensity, in other examples) determined by the processing module 30, such that the apoptosis rate approaches or achieves a target apoptosis rate. In 420, the processing module 30 generates a control instruction that causes the control unit 18 to control the probe manipulation device 16 to apply, according to the stimulus trajectory, the corresponding experimental stimulus $U_i$ to the biological probes 20. The probe manipulation device 16 can be configured to sequentially adjust the concentration of the bioactive agent according to the stimulus trajectory without any particular time control, or to do so in a timely controlled manner, for example such that a predefined time interval lapses between different concentrations of the bioactive agent corresponding to different, in particular consecutive, experimental stimuli of the stimulus trajectory.

If the processing module 30 determines at 416 that the number of cyclic repetitions of operations 402 to 406 corresponds to N or a multiple thereof, method 400 proceeds to operation 418, in which the processing module uses the sequence of measurement values or the sequence of measurement values and associated environmental conditions for fitting model parameters of a biological model for estimating values of the cellular parameter of the one or more biological probes as a function of the corresponding environmental condition and of the model parameters, corresponding to operation 312 of the method 300 illustrated in Fig. 5. The method then proceeds to operation 420, and then back to operation 402.

If the processing module determines, at 406, that the number of repetitions of operations 402 and 404 exceeds the number M, i.e. that the current stimulus trajectory has been completed, it goes on to operation 408, in which the processing module determines whether a convergence criterion of the regulatory task is fulfilled. In the embodiment illustrated in Fig. 6, the convergence criterion corresponds to a maximum number P of overall repetitions of operations 402 to 406, wherein P is greater than M, preferably a multiple of M, e.g. $100 \cdot M$. If this is the case, the method 400 is terminated at 412.

Otherwise, if condition 408 has a negative result, meaning that the convergence criterion is not fulfilled yet, the method 400 proceeds to 410. In 410, the processing module 30 determines an updated stimulus trajectory $U_1^{updated}, \ldots,$ $U_M^{updated}$. Examples of the determination of the updated stimulus trajectory shall be provided below.

After operation 410, the method 400 proceeds to operation 414, in which the processing module replaces the (previous) stimulus trajectory $U_1, \ldots, U_M$ by the updated stimulus trajectory $U_1^{updated}, \ldots, U_M^{updated}$ and goes back to operation 402 to restart the sequence of operations 402 to 420 for a further iteration as long as the convergence criterion evaluated in operation 408 is not fulfilled. Thus, when, in subsequent alterations, operation 420 is carried out, the experimental stimulus applied is an experimental stimulus of the corresponding updated stimulus trajectory $U_i^{updated}$.

Method 400 thereby achieves an optimal experimental set-up for estimating the model parameters. A detailed example of an application of method 400 is described below as Example 1.

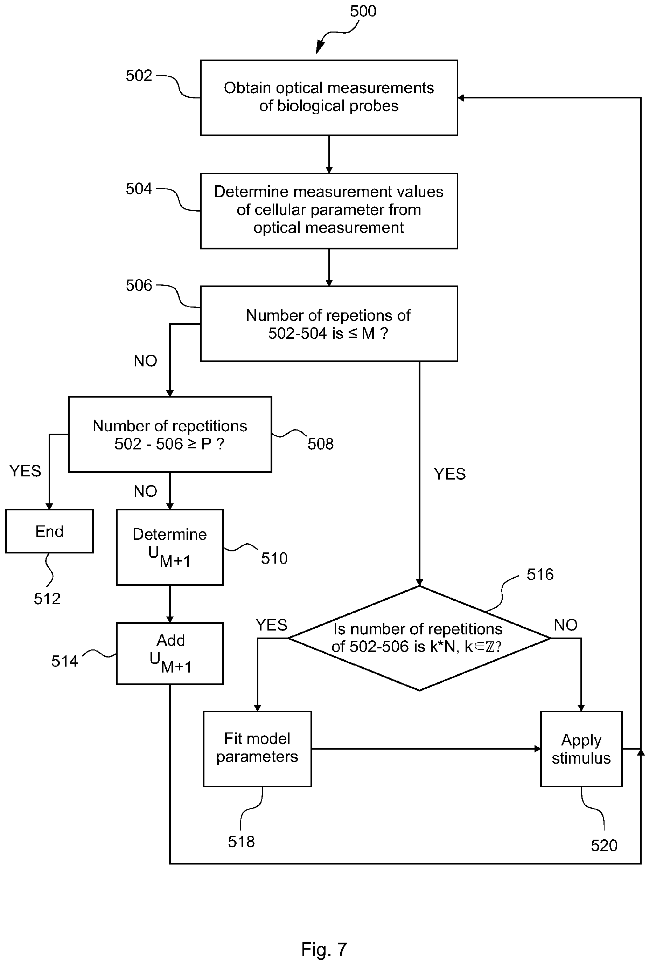

Fig. 7 is a flow diagram schematically illustrating a live-cell imaging method 500 according to an embodiment of the invention which may be implemented by a live-cell imaging system, for example by the live-cell imaging system 10 of Fig. 1 under the control of the processing module 30. The method 500 is a variation of method 400 illustrated in Fig. 6, wherein operations 502 to 508, 512, 516 and 518 respectively correspond to operations 402 to 408, 412, 416 and 418 of method 400 illustrated in Fig. 5.

However, according to method 500, when the processing module 30 determines negative outputs of conditions 506 and 508, the method 500 proceeds to 510, wherein the processing module 30 determines a subsequent experimental stimulus $U_{M+1}$ to be added to the stimulus trajectory.

The subsequent experimental stimulus is determined for setting a "significant environmental condition" of at least one of the biological probes to which it is applied. The "significant environmental condition" is defined as an environmental condition having a value greater than a first environmental condition and smaller than a second environmental condition. The first and second environmental conditions are defined as environmental conditions respectively set by a first experimental stimulus and a second environmental stimulus, wherein a variation of the cellular parameter as a function of the environmental condition is extremal, e.g. maximal or minimal, between the first and second environmental conditions. Thus, the "significant environmental condition" is defined to be in a region of the stimulus trajectory at which an extremal variation of the cellular parameter is determined. The subsequent experimental stimulus $U_{M+1}$ is defined as a function of the first and second experimental stimuli. A detailed example of an application of method 500, including an exemplary manner for determining the subsequent experimental stimulus, is described below as Example 2.

After the subsequent experimental stimulus $U_{M+1}$ is determined in operation 510, the method 500 proceeds to operation 514, wherein the probe manipulation device 16 applies the determined subsequent experimental stimulus $U_{M+1}$ to the corresponding biological probes 20, and then proceeds back to operation 502, as illustrated in Fig. 7.

Thus, according to method 500, a closed-loop can be defined for determining data points, fitting the model parameters and determining the next applied experimental stimulus (e.g. drug concentration).

The method 500 may allow for a more comprehensive characterisation of drug response curves, for example IC50 curves, which can be relevant for selecting drug candidates. Further, the method 500 allows determining drug response curves for cell proliferation and for cell death rates separately, without confusing the sources or effects thereof.

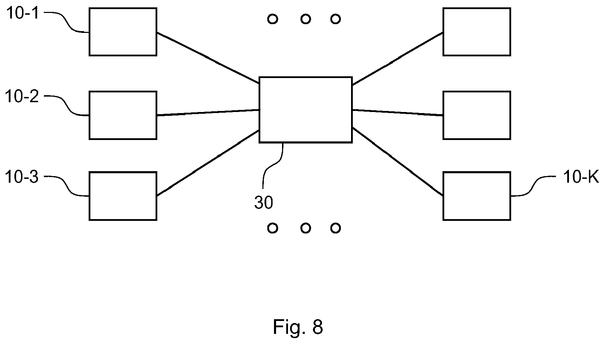

Fig. 8 schematically represents a plurality of live-cell imaging systems 10-1,...,10-K according to embodiments of the present invention. Each of the live-cell imaging systems 10-1,...,10-K is functionally connected to and controlled by a central processing module 30, for example via an internet connection between the central processing module 30 and each of the live-cell imaging systems 10-1,...,10-K. The processing module 30 is configured for controlling the probe manipulation devices of each of the live-cell imaging systems 10-1,...,10-K such that a different regulatory task, for example a different stimulus trajectory, can be applied to each of the live-cell imaging systems. The measurement values based on the optical measurements obtained by each of the live-cell imaging systems are all received and analysed by the processing module 30.

The processing module 30 can also be configured for controlling the probe manipulation devices of each of the live-cell imaging systems 10-1,...,10-K such that one regulatory task is distributed over the live-cell imaging systems. For example, a stimulus trajectory can be applied to the live-cell imaging systems 10-1,...,10-K, with a first part of the stimulus trajectory, for example a first number of experimental stimuli of the stimulus trajectory, being applied to a first live-cell imaging system 10-1, a second part of the stimulus trajectory, for example a first number of other experimental stimuli of the stimulus trajectory, being applied to a second live-cell imaging system 10-2, and so on. The measurement values based on the optical measurements obtained by each of the live-cell imaging systems can all be received and analysed by the processing module 30.

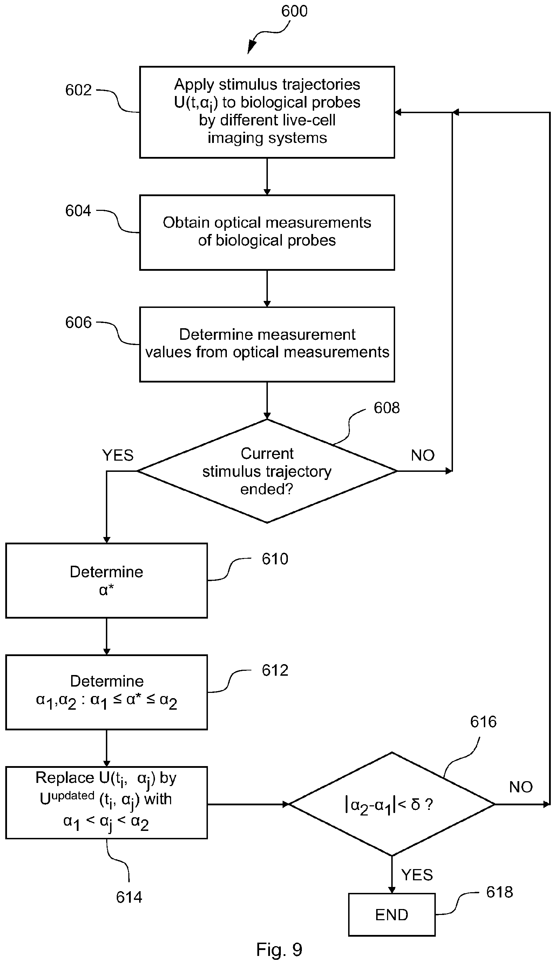

Fig. 9 is a flow diagram schematically illustrating a live-cell imaging method 600 according to an embodiment of the invention which may be implemented by a plurality live-cell imaging systems, for example by the live-cell imaging systems 10-1,...,10-K of Fig. 8 under the control of a processing module 30 via corresponding internet connections.

According to method 600, K stimulus trajectories $U_i^j$ (j=1,...,K), each with M experimental stimuli (i = 1,...,M) are applied, at 602, to K different sets of biological probes by the probe manipulation devices 16 of respective live-cell imaging systems 10-1,...,10-K. "Set of biological probes" refers herein to the biological probes that are monitored and effected upon by a given live-cell imaging system.

The different stimulus trajectories are defined by different values of a stimulus parameter. For example, different time-dependent stimulus trajectories can be defined as a function of a stimulation parameter $\alpha$. A first time-dependent stimulus trajectory $U(t_i, \alpha_1) = U(t_1, \alpha_1),...,U(t_M, \alpha_1)$ is applied to a first set of biological probes by a first live-cell imaging system 10-1, a second time-dependent stimulus trajectory $U(t_i, \alpha_2) = U(t_1, \alpha_2),...,U(t_M, \alpha_2)$ is applied to a second set of biological probes by a second live-cell imaging system 10-2, and so on until the K-th time-dependent stimulus trajectory $U(t_i, \alpha_K) = U(t_1, \alpha_K),...,U(t_M, \alpha_K)$ is applied to a set of biological probes by the K-th live-cell imaging system 10-K.

At 602, the processing module controls the probe manipulation devices 16 of the live-cell imaging systems 10-1,...,10-K to simultaneously apply a respective one of the stimulus trajectories $U(t_i, \alpha_1),...,U(t_i, \alpha_K)$ to the corresponding sets of biological probes.

In operation 604, the processing module 30 controls the imaging devices 12 of the live-cell imaging systems 10-1,..., 10-K to obtain optical measurements of the corresponding sets of biological probes. Operations 602 and/or 604 may overlap in time at least in part. In operation 606, the processing module 30 obtains the measurement values from the optical measurements obtained by the imaging device 12.

At 608, the processing module 30 determines whether a convergence criterion of a regulatory task is fulfilled, for example if a corresponding stimulus trajectory has been completed. The processing module 30 stores, for each obtained measurement value, the measurement value and the associated experimental stimulus.

If, at 608, it is determined that the stimulus trajectory has not been completed yet, the method 600 proceeds to operation 610, in which an extremal value $\alpha^*$ of the stimulus parameter $\alpha$ is determined by the processing module 30. The extremal value $\alpha^*$ corresponds to the stored experimental stimulus $U(t,\alpha_j)$, which, from all experimental stimuli of all stimulus trajectories applied to the biological probes, results in an extremal, e.g. maximal or minimal, measurement value.

The processing module then proceeds to determine, in 612, a first reference value $\alpha_1$, and a second reference value a2. The first and second reference values $\alpha_1$, $\alpha_2$ are determined such that $\alpha_1 \leq \alpha^* \leq \alpha_2$, preferably such that $\alpha_1 < \alpha^* < \alpha_2$. For example, if $\alpha^* = \alpha_j$, the first and second reference values can be defined as the values immediately preceding and immediately following the stimulus parameter $\alpha^*$, i.e. $\alpha_1=\alpha_{j-1}$ and $\alpha_2= \alpha_{j+1}$.

Then, in operation 614, the processing module 30 replaces the K different stimulus trajectories that are applied to the K different sets of biological cells by the K the updated stimulus trajectories $U^{updated}(t_i,\alpha_j)$ with i = 1, ..., M and j = 1, ..., K, respectively. The updated stimulus trajectories are respectively determined by a value of the stimulus parameter $\alpha$ lying between the first reference value $\alpha_1$, and the second reference value a2, i.e. for $\alpha_1 \leq \alpha_j \leq \alpha_2$ for all j.

The method 600 then proceeds to operation 616, in which the processing module determines whether a difference between the first and second reference values $|\alpha_2-\alpha_1|$ is smaller than a predefined threshold value $\delta$. If this is the case, the method 600 is terminated at 618. Otherwise, the method 600 goes back to operation 602 for a further iteration of method operations 602 to 616.

PRACTICAL EXAMPLES

EXAMPLE 1

Example 1 is an example of a live-cell imaging method 400 as illustrated in Fig. 5. the cellular parameter being measured or estimated is an apoptosis rate of CD95-receptor overexpressing HeLa cells (human cervical cancer cell line) present in the biological probes 20.

Apoptotic cells are identified by the processing module 30 using a correspondingly trained convolutional network algorithm that identifies dead cells by means of image segmentation on the basis of image local contrast using images of one of the biological probes 20.

The probe manipulation device 16 is configured for controlling the concentration of a bioactive agent in an experimental fluid and for perfusing the biological probes with the experimental fluid by means of a pump for pumping the experimental fluid, an output reservoir for collecting experimental fluid residues after they have flown in contact with the biological probes, and fluid conduits fluidly connecting each of the biological probes between the pump and the output reservoir. The bioactive agent is T4-CD95L, which favours cell death due to binding of the cell death ligand (CD95L), a bioactive agent that influences concentration of $CD_{95}L$ in the experimental fluid.

In the exemplary embodiment under consideration, a stimulus trajectory is defined by a time-dependent concentration of the $CD_{95}L$ death ligand as given by the function:

$$u(t)= U_0 \exp(-U_1 \cdot t) \qquad (1)$$

$U_0$ being the initial concentration of the death ligand at an initial time t=0, which can be, for example 500 ng/ml. The stimulus trajectory is hence defined by a sequence of concentrations of the CD95L death ligand corresponding to evaluations of the function u(t) of equation (1) at times corresponding to multiples of a predefined time interval $\Delta t$, i.e $U_i = u(i \cdot \Delta t)$ with I being a natural number, i=1, ..., M.

The used biological model is based on a set of three coupled ODEs and describes binding of the cell death ligand CD95L to cell death receptors CD95R, release of the ligand, activation of an effector caspase C* by active death receptors, and inactivation of the effector caspase C*:

$$\frac{d[CD95R]}{dt} = -k_{on}[CD95L][CD95R] + k_{off}[CD95R^*] \qquad (2)$$

$$\frac{d[CD95R^*]}{dt} = k_{on}[CD95L][CD95R] - k_{off}[CD95R^*] \qquad (3)$$

$$\frac{d[C^*]}{dt} = k_{act}\frac{[CD95R^*]^h}{K^h+[CD95R^*]^h} - k_{inact}[C^*] \qquad (4)$$

[CD95R] is the concentration of the cell death receptor CD95R, [CD95L] is the concentration of the cell death ligand CD95L, [CD95R*] is the concentration of active CD95R cell death receptors, and [C*] is the concentration of active effector caspase. The biological model is further defined by model parameters $k_{on}$, $k_{off}$, $k_{act}$, and $k_{inact}$, which are kinetic parameters respectively describing binding, unbinding, activation of the effector caspase and inactivation of the active effector caspase, and by the Hill-type function parameters $h$ and $K$, which are used for modelling effector caspase activation. According to this biological model, a number or fraction of dead cells can be associated with a fraction of active effector caspase. Thus, the biological model defined by equations (2) to (4) provides estimated values of the cellular parameter (i.e. here a fraction of apoptotic cells) as a function of the environmental condition defined by the experimental stimuli (concentration of the cell death ligand [CD95L]) and the model parameters $k_{on}$, $k_{off}$, $k_{act}$, $k_{inact}$, $h$ and $K$.

The updated stimulus trajectory is determined by the processing module such that a covariance of the one or more model parameters as a function of the stimulus trajectory is minimised. For a parameter vector $\theta$ (e.g. $\theta = [k_{on}, k_{off}, k_{act}, k_{inact}, h, K]^T$), and an estimate of parameters obtained from model fitting $\hat{\theta}$, the covariance matrix of the model parameters can be defined as an expected value $C_\theta = E((\hat{\theta} - \theta)(\hat{\theta} - \theta)^T)$. For example, the processing module can be configured to calculate a sensitivity matrix $S_{t_i} = \frac{dy(t_i)}{d\theta}$ defined for a function $y\{x(t_i),u(t_i),\theta\}$, with x being a vector containing the concentrations of model quantities of the biological model given by equations (2) to (4), $x = [[CD95R], [CD95R^*], [C^*]]^T$ at time points $t_i$, with i = 1, ..., M. Using an estimate of the covariance matrix $C_y$ defined for the optical measurements obtained by the imaging device 12 and sensitivities at different time points $S_{t_i}$, the so-called Fisher information matrix $F = \sum_{t_i} S_{t_i}^T C_y^{-1} S_{t_i}$ can be calculated. A lower bound for elements of the covariance matrix of the model parameters can be obtained from $C_\theta \geq F^{-1}$. Thereby, a measure for the covariance of the one or more model parameters as a function of the stimulus trajectory can be estimated.

Thus, the processing module can be configured to determine the updated stimulus trajectory such that the covariance of the one or more model parameters as a function of the stimulus trajectory, i.e. the trace or the determinant of $C_\theta$ or of $F^{-1}$ be minimised to obtain more accurate estimates of model parameters. The updated stimulus trajectory is then obtained (in operation 410 of the method illustrated in Fig. 6) as the solution to this optimisation problem. The updated stimulus trajectory sets experimental conditions according to the regulatory task of reducing the confidence interval of the estimated model parameters $k_{on}$, $k_{off}$, $k_{act}$, $k_{inact}$, $h$ and $K$, thereby improving the accuracy and reliability thereof.

When the convergence criterion defined for condition 408 is fulfilled, an improved estimation of the model parameters $k_{on}$, $k_{off}$, $k_{act}$, $k_{inact}$, $h$ and $K$ is obtained. The experimental design has up to then been designed in an optimised manner by choosing experimental conditions such that a confidence of the model parameters be minimised. Thus, an improved or more reliable version of the biological model defined by equations (2) to (4) is obtained in an efficient and automated manner based on closed-loop live-cell imaging.

EXAMPLE 2

Example 2 is a detailed example of a live-cell imaging method 500 as illustrated in Fig. 7. The cellular parameters being measured are a number of apoptotic cells A and a number of living cells L in each of the biological probes 20.

The probe manipulation device 16 is configured for controlling a concentration d of a chemotherapeutic drug in an experimental fluid and for perfusing the biological probes with the experimental fluid, thereby respectively setting environmental conditions thereof. The stimulus trajectory is initially defined here as a sequence of (initially three) drug concentrations $U_1 = d_{max}/100$, $U_2 = d_{max}/10$ and $U_3 = d_{max} = U_M$.

The biological model being used is a model that describes the number of living cells L and the number of dying cells A, a growth rate $\theta_g$ and an apoptosis rate $\theta_a$ as a function of the chemotherapeutic drug concentration d and of a set of model parameters $k_g$, $k_d$, $K_i$, $K_d$, $h$ and $l$:

$$\frac{dL}{dt} = \left(\theta_g - \theta_a\right)L, \qquad (5)$$

$$\frac{dA}{dt} = \theta_a L, \qquad (6)$$

$$\theta_g(d) = k_g \frac{1}{1 + \left(\frac{d}{K_i}\right)^h} \qquad (7)$$

$$\theta_a(d) = k_d \frac{\left(\frac{d}{K_d}\right)^l}{1 + \left(\frac{d}{K_d}\right)^l} \qquad (8)$$

where $k_g$ denotes a maximal speed of growth, $k_d$ denotes a maximal speed of cell death, $K_i$ denotes a drug concentration at which the proliferation rate is decreased to half its maximal value, $K_d$ denotes the chemotherapeutic drug concentration resulting in the half-maximal cell death rate, and $h$ and $l$ are Hill-function parameters describing the steepness of the involved sigmoidal curves.

In method 500, operation 518 comprises (re)fitting the model parameters $k_g$, $k_d$, $K_i$, $K_d$, $h$ and $l$, to the set of data points (measurement values, i.e. values of A and L + environmental conditions, i.e. values of d) previously collected and stored by the processing module 30 based on a previous estimation of the growth and cell death rates $\theta_g$ and $\theta_a$. For parameter estimation, the biological model can for example be fitted in a two-step procedure: first, a current sequence of measurement values of L and A corresponding to optical measurements obtained by the imaging device 12 at a corresponding drug concentration $d$ can fitted based on equations (5) and (6) of the biological model to determine the growth and apoptosis (cell death) rates $\theta_g$ and $\theta_a$. Then, equations (7) and (8) of the biological model can be fitted to the obtained values of $\theta_g$ and $\theta_a$ to determine the parameters $k_g$, $k_d$, $K_i$, $K_d$, $h$ and $l$. In general, the model parameter $K_d$ is of major interest to describe the effectiveness of the chemotherapeutic drug against cancer cells and should therefore be accurately determined.

In some examples, the probe manipulation device 16 may be configured for simultaneously applying each of the different drug concentrations to different biological probes, for example different biological probes monitored and influenced by the same live-cell imaging system (e.g. different of the well plates shown in Fig. 3) or different biological probes monitored and influenced by different live-cell imaging systems (e.g. live-cell imaging systems 10-1 to 10-K shown in Fig. 8).

The different live-cell imaging systems can be differently located, remote from each other, and centrally controlled by a common processing module, for example via an internet connection, as in the example illustrated in Fig. 8.

In operation 510 of method 500 illustrated in Fig. 7, the processing module determines whether the variation of the apoptosis rate $\theta_a$ is larger between the drug concentrations of the stimulus trajectories $U_1$ and $U_2$ or between $U_2$ and $U_3$. An iteration scheme can be defined, for example, using a comparative parameter E that describes the difference of apoptosis rates $\theta_{a,i}$ and $\theta_{a,j}$ at two different drug concentrations $d_i$ and $d_j$ with $d_i < d_j$:

$$E_{i,j} = k_d\left(\theta_{a,j} - \theta_{a,i}\right) \tag{9}$$

In an iteratively sequence based on the three last applied drug concentrations $U_{M-2} < U_{M-1} < U_M$ with $n \geq 3$, the subsequent experimental stimulus $U_{M+1}$ to be applied can then be defined, in operation 510 of method 500 illustrated in Fig. 7, as the mean of two of the three last applied $\log_{10}$-scaled concentrations:

$$U_{M+1} = \begin{cases} 10^{\frac{1}{2}\left(log_{10}(U_{M-1})+log_{10}(U_{M-2})\right)} & if\ E_{M-2,M-1} \geq E_{M,M-1} \\ 10^{\frac{1}{2}\left(log_{10}(U_{M})+log_{10}(U_{M-1})\right)} & if\ E_{M-2,M-1} < E_{M,M-1} \end{cases} \tag{10}$$

A sequence of iteration steps consisting of (I) automatically applying a drug concentration according to the current stimulus trajectory, (II) obtaining optical measurements of the biological probes and corresponding measurement values, (III) fitting the model parameters and (IV) determining a subsequent experimental stimulus to be applied based on the scheme defined by equations (9) and (10) can be applied until a certain termination criterion is fulfilled. This termination criterion can be defined as a maximal number of iterations of steps (I) to (IV) or based on the comparative parameter E defined in equation (9). For example, the method can be terminated when the number of iterations of steps (I) to (IV) reaches or exceeds a predefined threshold $M_{max}$, or when the comparative parameter $E_{M,M-1}$, falls below a certain predefined threshold $E_{min}$. Thereby, the parameter estimation of the model parameters $k_g$, $k_d$, $K_i$, $K_d$, $h$ and $l$, in particular of the most relevant model parameter $K_d$, is efficiently optimised by iteratively selecting most informative data points and hence concentrating data acquisition in relevant regions of the variable space.

As an alternative to the iteration scheme defined by equations (9) and (10), other iterative schemes can be used.

EXAMPLE 3

Example 3 is a further example of a live-cell imaging method according to the present invention, for example according to the method 600 illustrated in Fig. 9. According to Example 3, the cellular parameter being measured or estimated is an apoptosis rate in each of the biological probes 20.

It is known that the activity of CD95 cell death receptors stimulated by the ligand T4-CD95L follows an inverse bell shape, depending on the dose or concentration of the ligand. An increased concentration of T4-CD95L results in an increased receptor activity and faster cell death. However, after a certain peak concentration is exceeded, further increasing the concentration of the ligand T4-CD95L results in decreased receptor activity and hence decreased cell death. The observation that cell viability is lowest for intermediate cell death ligand concentrations has important implications for efficiently using a cell death ligand, which is injected into the cellular compartment and thereafter eliminated from the compartment, to efficiently induce apoptosis in a population of cells. For a pre-defined total amount of cell death ligand, injecting the ligand too slow will not result in sufficient activation of CD95 receptors, whereas, injecting the ligand too fast will result in less cell death as in case of an intermediate injection speed. The aim of the application of the method according to this example of closed-loop live-cell imaging is finding an optimal injection speed that maximizes cell death in an automated manner.

The aforesaid optimal injection speed of the T4-CD95L, at which a maximal fraction of cells undergoing apoptosis is achieved, can be determined by means of method 600 illustrated in Fig. 9. To this end, the stimulus trajectory is iteratively optimized as described in the following.

According to an example, different stimulus trajectories applied to different biological probes 20 are based on the following ODE model that simulates a ligand concentration L in the blood serum of a patient after intravenous administration of a drug, and a concentration $L_m$ of the drug at the site of a tumour within the body of the patient:

$$\frac{dL}{dt} = -k_{in}L \tag{11}$$

$$\frac{dL_m}{dt} = k_{in}L - k_{out}L_m \tag{12}$$

wherein the drug transport to the site of the tumour is described by the injection rate $k_{in}$ and drug removal from the site of the tumour is described by the parameter $k_{out}$.

The solution to the coupled ODEs (11) and (12) that describe a pulse of transient ligand accumulation and removal is:

$$L_m(t) = \frac{k_{in}L_0}{k_{out}-k_{in}}\left(exp(-k_{in}t) - exp(-k_{out}t)\right) \tag{13}$$

Concentration trajectories described by equation (13) with different values for the injection rate $k_{in}$ can be applied by the probe manipulation device 16 of a live-cell imaging system 10 to simulate biological processes at corresponding biological probes according to method 600.

In operation 602, M different sets of biological probes are treated with stimulus trajectories $L_m(t,k_{in,i})$ at different injection rates $k_{in,i}$ for $i$=1, ..., M (i.e. wherein $k_{in,i}$ corresponds here to the stimulus parameter $\alpha_i$ as described in Fig. 9).

The different injection rates $k_{in,i}$ can be chosen to be

$$k_{in,i} = 10^{\left[(log_{10}(k_{in,max,0})-log_{10}(k_{in,min,0}))\frac{i-1}{M-1}+log_{10}(k_{in,min,0})\right]} \tag{14}$$

with $k_{in,min,o}$ and $k_{in,max,o}$ being, respectively, the minimum and the maximum injection rates supported by the probe manipulation device 16.

In operations 604 and 606, numbers of living and dead cells of the biological probes are segmented from optical measurements obtained by the imaging device 12 with a pre-defined time interval between subsequent optical measurements of e.g. 1 hour and the cell death rate $\theta_a$ is estimated, for example using the biological model described for Example 2, i.e. using equations (5) to (8), fitted to the measurement values obtained by the processing module 30.

In operation 610, after the M sets of biological probes have been treated with the respective stimulation trajectories, the injection rate $k_{in,m}=k_{in}*=\alpha*$ resulting in the maximum cell death rate is determined by the processing module 30.

The first and second reference values for this initial iteration are then determined, at operation 612, as $k_{in,m-1}$ and $k_{in,m+1}$ according to equation (14).

Accordingly, in each of the subsequent iteration steps with indices $l$ = 1... $Q$, the new minimum and maximum injection rates for the updated stimulus trajectories are respectively determined as a function of the injection rate $k_{in,m}=k_{in}*$ resulting in the maximum cell death rate in the previous iteration, as:

$$k_{in,min,l} = 10^{\left[(log_{10}(k_{in,max,l-1})-log_{10}(k_{in,min,l-1}))\frac{m-2}{M-1}+log_{10}(k_{in,min,l-1})\right]} \tag{15}$$

$$k_{in,max,l} = 10^{\left[(log_{10}(k_{in,max,l-1})-log_{10}(k_{in,min,l-1}))\frac{m}{M-1}+log_{10}(k_{in,min,l-1})\right]} \tag{16}$$

The stimulus trajectories $L_{m,i}(t)$ are then replaced, in operation 614, by updated stimulus trajectories defined by the subsequent set of injection rates comprised between $k_{in,m-1}$ and $k_{in,m+1}$, with $j$ = 1 ... $M$:

$$k_{in,j} = 10^{\left[(log_{10}(k_{in,max,l})-log_{10}(k_{in,min,l}))\frac{j-1}{M-1}+log_{10}(k_{in,min,l})\right]} \tag{17}$$

Operations 602 to 614 are then repeated as long as, in 616, the processing module does not determine that $|k_{in,max,l} - k_{in,min,l}|$ (cf. $|\alpha_2,-\alpha_1|$ is below a threshold value $\delta$, or, additionally or alternatively, in case the maximum number of overall iterations $l = Q$ is reached.

Thus, the method 600 allows automatically determining dose-response curves in an efficient manner by enabling feedback between an experimental variable (cf. drug concentration) and a cellular parameter monitored by a live-cell imaging system, based on a pharmacokinetic model describing the drug concentration at the site of a tumour.

In related examples, stimulus trajectories can be optimised for more than one stimulus parameter.

**Claims**

1. A live-cell imaging method for acquiring experimental data of one or more biological probes (20) comprising:

   i. obtaining, by an imaging device (12), at least one optical measurement of the one or more biological probes (20);
   ii. determining, by a processing module (30), at least one measurement value of a cellular parameter of the one or more biological probes from the at least one optical measurement;
   iii. determining, by the processing module (30), whether the at least one measurement value satisfies a convergence criterion of a regulatory task; and
   iv. applying, by a probe manipulation device (16), at least one experimental stimulus to the one or more biological probes (20) according to the regulatory task, if the convergence criterion is not fulfilled, wherein the experimental stimulus sets an environmental condition of the biological probe to which the experimental stimulus is applied, wherein the regulatory task defines a target evolution of the one or more biological probes (20).

2. The method of claim 1, wherein the convergence criterion is fulfilled when the determined measurement value of the cellular parameter corresponds to a target value of the cellular parameter defined by the regulatory task within a predefined tolerance.

3. The method of claim 1 or 2, wherein the regulatory task defines the target value of the cellular parameter such that: the cellular parameter or a derivative thereof is kept constant, the cellular parameter follows a predefined parameter trajectory, and/or a biological process depending on the cellular parameter of the corresponding probe achieves a target state.

4. The method of one of the preceding claims, wherein the method further comprises: recording a sequence of measurement values and associated environmental conditions corresponding to a number of cyclic repetitions of steps i to iii or i to iv and

   v. fitting one or more model parameters of a biological model for the one or more biological probes (20) to the sequence of measurement values and environmental conditions, wherein the biological model provides estimated values of the cellular parameter of the one or more probes as a function of the corresponding environmental condition and of the model parameters; wherein the number of cyclic repetitions preferably is 5, 10, 15, 25, 50, 100, or 1000.

5. The method of claim 1 or 4, wherein the regulatory task defines a target environmental condition of the biological probe to which the experimental stimulus is applied according to a stimulus trajectory, wherein the stimulus trajectory determines a sequence of experimental stimuli;
   wherein fitting the one or more model parameters of the biological model preferably further comprises:

   determining an updated stimulus trajectory; and
   applying to at least one of the one or more biological probes (20), by the probe manipulation device (16), at least one of the experimental stimuli of the updated stimulus trajectory;

   wherein the updated stimulus trajectory is determined such that a covariance of the one or more model parameters as a function of the stimulus trajectory is minimised.

6. The method of claim 5 wherein fitting the one or more model parameters of the biological model further comprises:

   - determining a subsequent experimental stimulus of the stimulus trajectory for setting an significant environmental condition of at least one of the one or more biological probes (20), the significant environmental condition having a value greater than a first environmental condition and smaller than a second environmental condition, wherein the first environmental condition is set by a first experimental stimulus of the stimulus trajectory and the second environmental condition is set by a second experimental stimulus of the stimulus trajectory, wherein a variation of the cellular parameter as a function of the environmental condition is extremal between the first and second environmental conditions with respect to the environmental conditions set by the stimulus trajectory;

and
- applying to said at least one of the one or more biological probes (20), by the probe manipulation device (16), the subsequent experimental stimulus

wherein the subsequent experimental stimulus is preferably determined as a function of the first and second experimental stimuli.

**7.** The method of any of the preceding claims, wherein different regulatory tasks defining different stimulus trajectories are applied to different biological probes (20), wherein the optical measurements of the different biological probes (20) are sequentially or simultaneously obtained by one imaging device (12) or by different imaging devices (12) wherein the different stimulus trajectories are preferably defined by different values of at least one stimulus parameter, wherein the method further comprises:

- determining an extremal value of the at least one stimulus parameter, wherein the extremal value of the at least one stimulus parameter corresponds to an experimental stimulus of the different stimulus trajectories for which an extremal measurement value is determined;
- determining first and second reference values of the stimulus parameter, wherein the first reference value is smaller than the extremal value and the second reference value is greater than the extremal value;
- applying to at least one of the one or more biological probes (20), by the probe manipulation device (16), respective updated stimulus trajectories determined by different values of the at least one stimulus parameter ranging between the first and second reference values.

**8.** The method of any of the preceding claims, wherein determining the measurement value of the cellular parameter comprises classifying, counting and/or identifying cells in the corresponding biological probe with respect to the cellular parameter wherein the cells are preferably classified as living or dead; and/or
wherein the cells are classified, counted and/or identified by a neural network algorithm trained for classifying, counting and/or identifying cells of the one or more biological probes (20) based on one or more optical measurements with respect to the cellular parameter; and/or
wherein the cellular parameter comprises one or more of cell number, living cell number, living cell fraction, dead cell number, dead cell fraction, cell proliferation rate, cell death rate, cell division rate, cell differentiation rate, cell exocytosis rate, cell endocytosis rate, cell size, cell dimensions, cell adherence area, beating frequency, cell depolarization rate, and drug concentration.

**9.** A live-cell imaging system (10) for acquiring experimental data of one or more biological probes (20) comprising:

- an imaging device (12) for obtaining at least one optical measurement of the one or more probes;
- a probe manipulation device (16) for applying at least one experimental stimulus to the one or more probes, wherein the experimental stimulus sets an environmental condition of the biological probe to which the experimental stimulus is applied; and
- a control unit (18) configured for controlling the operation of the imaging device (12) and the probe manipulation device (16) based on control instructions received over a functional connection (40), wherein the control unit (18) is configured for:
outputting over the functional connection (40) information comprising the obtained at least one optical measurement or related thereto; and controlling the probe manipulation device (16) to apply the at least one experimental stimulus to the one or more probes according to the control instructions.

**10.** The live-cell imaging system of claim 9, wherein the imaging device (12) comprises an optical device (126), in particular one or more of a microscope, a digital camera, a CCD, one or more mirrors, one or more deflectors and/or one or more focusing lenses; and/or
wherein the imaging device (12) or the optical device (126) is movable for scanning the one or more probes, and wherein the control unit (18) is further configured for controlling a movement of the imaging device (12) or the optical device (126); and/or wherein the imaging device (12) comprises a housing (121) enclosing at least some of the remaining components of the imaging device (12);
wherein the housing (121) preferably comprises a cover plate (122), a bottom plate (132) and at least a lateral wall (124) extending between the cover plate (122) and the bottom plate (132),
wherein the cover plate (122) preferably is at least partly transparent and is configured for supporting the one or more biological probes (20) and/or one or more probe carriers containing the one or more biological probes (20), and/or

wherein the housing (121) preferably comprises a metallic bottom plate (132).

11. The live-cell imaging system of any of claims 9 to 10, further comprising a reflective element (50) for directing illumination light to and/or through the one or more biological probes (20) and an illumination light source for generating the illumination light for illuminating the one or more biological probes (20) for obtaining the at least one optical measurement by the imaging device (12).

12. The live-cell imaging system of any of claims 9 to 11, wherein the probe manipulation device (16) comprises a perfusion device for perfusing the one or more biological probes (20) with an experimental fluid; and/or
wherein the probe manipulation device (16) comprises a light source, preferably an LED, for emitting experimental light on the one or more probes.

13. A processing module (30) connectable to the functional connection (40) of a live-cell imaging system (10) according to any of claims 19 to 26, wherein the processing module (30) is configured for:

- receiving, over the functional connection (40), information comprising the at least one optical measurement or related thereto;
- determining, based on said information, at least one measurement value of a cellular parameter of the one or more probes;
- determining whether the at least one measurement value satisfies a convergence criterion of a regulatory task;
- if the convergence criterion is not satisfied, determining at least one experimental stimulus, wherein the at least one experimental stimulus sets an environmental condition of the biological probe to which the experimental stimulus is applied so as to improve fulfilment of the convergence criterion; and
- sending control instructions through the functional connection (40) for controlling the probe manipulation device (16) to apply the determined at least one experimental stimulus to at least one of the one or more biological probes (20).

14. The processing module (30) of claim 13, further configured for controlling the live-cell imaging system so as to implement the method defined in any of claims 1 to 8.

15. A digital storage device comprising executable code which, when executed by a processor, configures the processor for operating as a processing module (30) according to claim 13 or 14.

Fig. 1

Fig. 2

Fig. 3

Fig. 4

300

302

Obtain optical measurements
of biological probes

304

Determine measurement values
of cellular parameter from
optical measurement

YES

Determine whether convergence
criterion of regulatory task
is fulfilled

306

308

YES

Is number of repetitions
of 302-306 k*N, k∈ℤ?

NO

Fitting model
parameters

Apply experimental
stimulus to biological
probes

312

310

Fig. 5

400

402 — Obtain optical measurements
of biological probes

404 — Determine measurement values
of cellular parameter from
optical measurement

406 — Number of repetions of
402-404 is ≤ M ?

NO

Number of repetitions
402 - 406 ≥ P ? ~808 408

YES

NO

End

412

Determine
updated
$U_i^{updated}$ ~410 410

$U_i \longrightarrow U_i^{updated}$

414

YES

Is number of repetitions
of 402-406 is k*N, k∈ℤ? 416

NO

YES

Fit model
parameters

418

Apply
stimulus

420

Fig. 6

Fig. 7

Fig. 8

600

602 — Apply stimulus trajectories
$U(t,\alpha_j)$ to biological probes
by different live-cell
imaging systems

604 — Obtain optical measurements
of biological probes

606 — Determine measurement
values from optical measurements

608

YES  Current
stimulus trajectory
ended?  NO

610  Determine
$\alpha^*$

612  Determine
$\alpha_1, \alpha_2 : \alpha_1 \leq \alpha^* \leq \alpha_2$

616

Replace $U(t_i, \alpha_j)$ by
$U^{updated}(t_i, \alpha_j)$ with
$\alpha_1 < \alpha_j < \alpha_2$  $|\alpha_2 - \alpha_1| < \delta$ ?  NO

614

YES

END  618

Fig. 9

Europäisches
Patentamt

European
Patent Office

Office européen
des brevets

# EUROPEAN SEARCH REPORT

Application Number

EP 19 20 4932

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/060214 A2 (UNIV CALIFORNIA [US]; HO CHIH-MING [US]; WONG PAK KIN [US]) 8 June 2006 (2006-06-08) * paragraphs [0055] - [0081]; figure 1 * ----- | 1-15 | INV. G01N33/50 G01N35/00 G06K9/00 G06T7/00 |
| X | US 2014/273045 A1 (LUDWIG LESTER F [US]) 18 September 2014 (2014-09-18) | 1-3,9, 13-15 | |
| A | * paragraphs [0095] - [0133], [0158] * ----- | 4-8,12 | |
| A | WO 01/42786 A2 (CELLOMICS INC [US]; SAMMAK PAUL [US] ET AL.) 14 June 2001 (2001-06-14) * pages 15-16; figures 7, 8 * ----- | 1,9-15 | |
| A | EP 3 388 832 A1 (SYSMEX CORP [JP]) 17 October 2018 (2018-10-17) * paragraphs [0088] - [0091]; figure 4 * ----- | 1,9-11, 13,15 | |

| TECHNICAL FIELDS SEARCHED (IPC) |
|---|
| G01N G06T G06K |

The present search report has been drawn up for all claims

| Place of search | Date of completion of the search | Examiner |
|---|---|---|
| Berlin | 8 May 2020 | Dydenko, Igor |

EPO FORM 1503 03.82 (P04C01)

## ANNEX TO THE EUROPEAN SEARCH REPORT
## ON EUROPEAN PATENT APPLICATION NO.

EP 19 20 4932

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2020

| Patent document cited in search report | | Publication date | Patent family member(s) | | Publication date |
|---|---|---|---|---|---|
| WO 2006060214 | A2 | 08-06-2006 | US 2009075360 A1 | | 19-03-2009 |
| | | | US 2013130360 A1 | | 23-05-2013 |
| | | | US 2014363805 A1 | | 11-12-2014 |
| | | | US 2016326482 A1 | | 10-11-2016 |
| | | | WO 2006060214 A2 | | 08-06-2006 |
| US 2014273045 | A1 | 18-09-2014 | NONE | | |
| WO 0142786 | A2 | 14-06-2001 | AU 2073801 A | | 18-06-2001 |
| | | | US 2001041347 A1 | | 15-11-2001 |
| | | | WO 0142786 A2 | | 14-06-2001 |
| EP 3388832 | A1 | 17-10-2018 | CN 108761054 A | | 06-11-2018 |
| | | | EP 3388832 A1 | | 17-10-2018 |
| | | | JP 2018179709 A | | 15-11-2018 |
| | | | US 2018292385 A1 | | 11-10-2018 |

EPO FORM P0459

For more details about this annex : see Official Journal of the European Patent Office, No. 12/82